# EUROPÄISCHE PATENTSCHRIFT

(11) **EP 0 547 446 B1**
(45) Veröffentlichungstag und Bekanntmachung des Hinweises auf die Patenterteilung: **29.04.1998**
(21) Anmeldenummer: 92120661.1
(22) Anmeldetag: 03.12.1992
(51) Int. Cl.: C12N 15/38, C12N 15/01, C07K 14/00, A61K 39/245, G01N 33/569

(54) **Impfstoffe auf Basis geänderter Boviner Herpesviren Typ 1**
Vaccines based on modified bovine herpesvirus type I
Vaccins basés sur l'herpesvirus bovin type I modifié

(30) Priorität: 16.12.1991 DE 4141400
(43) Veröffentlichungstag der Anmeldung: 23.06.1993
(73) Patentinhaber: BAYER AG, 51368 Leverkusen (DE)
(72) Erfinder: Floss, Georg, Dr., W-5000 Köln 60 (DE); Strube, Walter, Dr., W-5000 Köln 40 (DE); Thein, Peter, Prof. Dr. Dr. habil., W-8064 Oberzeitlbach (DE); Keil, Günther, Dr., W-7400 Tübingen (DE)

(56) Entgegenhaltungen:
- EP-A- 0 316 658
- VACCINE, Bd. 8, Nr. 4, August 1990, GUILDFORD GB, Seiten 358-368; S. VAN DRUNEN LITTEL-VAN DEN HURK: 'Epitope specificity of the protective immune response induced by individual bovine herpesvirus-1 glycoproteins'
- JOURNAL OF VIROLOGY, Bd. 64, Nr. 10, Oktober 1990, Seiten 5132 - 5142; S. TIKOO ET AL: 'Molecular cloning, sequencing, and expression of functional bovine herpesvirus 1 glycoprotein gIV in transfected bovine cells'

## Beschreibung

Die vorliegende Erfindung betrifft Impfstoffe auf Basis von Bovinen Herpesviren des Typs 1 (BHV-1) die Änderungen in Bereichen ihres Genoms enthalten, die für nicht-essentielle Teile essentieller Proteine kodieren. Mit Hilfe dieser Impfstoffe können geimpfte von nicht-geimpften Rindern unterschieden werden. Die Erfindung betrifft ferner Verfahren zur Isolierung und Herstellung der geänderten BHV-1 Stämme, Isolierung und Herstellung der geänderten Proteine und Peptide.

Die Infektion mit dem Bovinen Herpesvirus Typ 1 (BHV-1) manifestiert sich bei Rindern hauptsächlich in den Organen des Respirationstraktes und des Genitaltraktes, aber auch im peripheren und zentralen Nervensystem. Die hierdurch induzierten Krankheitsbilder werden beschrieben als Infektiöse Bovine Rhinotracheitis, Infektiöse Bovine Vulvovaginitis und Infektiöse Pustulöse Balanophosthitis. Daneben werden auch beobachtet Konjunktivitis, Orchitis, Endometritis, Mastitis, Aborte oder Meningoencephalitis. Nach der Primärinfektion kann BHV-1 im infizierten Tier lebenslang latent persistieren. Die Zellen des Nervensystems, aber auch das Epithelial-Oswebe, das lymphozytäre Gewebe, Makrophagen und Lymphozyten werden als Orte der Virus-Persistenz diskutiert. Als Folge von Reaktivierungen dieser latenten Infektionen wechseln sich Phasen mit klinischer Manifestation mit Virusausscheidung und Phasen unauffälliger Symptomatik mit oder ohne Erregerausscheidung ab. Belastungen des latent infizierten Organismus wie Streß, Immunsuppression oder Sekundärinfektion sind die Ursachen einer Reaktivierung.

Aufgrund der schweren wirtschaftlichen Schäden (wie beispielsweise Aufzuchtverluste, Abfall der Milchleistung, Rückgang der Befruchtungsquote, Sterilität und Aborte), die durch die BHV-1 assoziierten Krankheiten weltweit verursacht werden, haben prophylaktische Bekämpfungsmaßnahmen eine große Bedeutung. Neben der durch die Krankheiten verursachten Schäden haben die indirekten Folgen einer BHV-1-Infektion eine große Bedeutung. So dürfen BHV-1 verdächtige Rinder nur sehr begrenzt national und international gehandelt werden.

Für die prophylaktische Bekämpfung der BHV-1-assoziierten Krankheiten stehen Lebendimpfstoffe auf Basis attenuierter BHV-1-Stämme, inaktiverte Impfstoffe sowie sogenannte Spaltvakzinen, auf Basis gereinigter Virus-Proteine zur Verfügung. In derzeit laufenden Bekämpfungsmaßnahmen zur Eradikation von BHV-1 bzw. zur Sanierung von BHV-1-infizierten Bestanden und beim Tierexport werden Rinder als mögliche Virus-Träger anhand des Nachweises BHV-1-spezifischer Antikörper identifiziert.

Die Unterscheidung BHV-1-geimpfter Rinder von BHV-1-infizierten Rindern, die Eradikations- und Sanierungsmaßnahmen wesentlich effektiver gestalten könnte, ist derzeit nicht möglich.

Auf anderen Gebieten ist eine solche Unterscheidung geimpfter von infizierten Tieren möglich. Es sind bereits Impfstoffe für Schweine zur Prophylaxe der klinischen Manifestation der Infektion mit dem Aujeszky Virus (Procines Herpesvirus 1) entwickelt worden, die eine Differenzierung geimpfter und infizierter Tiere erlauben. Diese Vakzinen beruhen auf Virusstämmen, denen Proteine ganz oder in Teilen fehlen, die zur Virusvermehrung nicht notwendig sind, sogenannte nicht-essentielle Virusproteine, oder sie basieren auf einzelnen, gereinigten Virusproteinen. Serologische Nachweisverfahren zur Detektion spezifischer Antikörper, die gegen die in der Vakzine fehlenden Virusproteine gerichtet sind, erlauben eine Identifizierung Aujeszky Virusinfizierter Schweine und die Differenzierung zu Tieren, die gegen diesen Erreger immunisiert wurden.

Eine in EP-OS 316 658 beschriebene Vakzine soll die Unterscheidung geimpfter und infizierter Rinder erlauben. Es wird eine über rekombinante Technologie hergestellte BHV-1-Mutante beschrieben, die aufgrund einer Mutation (Deletion oder Insertion) im Gen für das nicht-essentielle Struktur-Glykoprotein gIII dieses Glykoprotein nicht enthält.

Einzelnen, viralen Proteinen können bestimmte biologische Funktionen zugeordnet werden. Das Fehlen von Protein-Komponenten in Viren, wie beispielsweise von Glykoproteinen, bedingt konsequenterweise das Ausbleiben von biologischen Funktionen dieser Viren. So ist das nicht-essentielle Struktur-Protein gIII des Aujeszky Virus bei der Immunantwort gegen das Aujeszky Virus bedeutend beteiligt. Speziell zelluläre Abwehrmechanismen, die bei der immunologischen Abwehr gegen Herpesviren eine hervorragende Rolle innehaben, sind gegen das Protein gIII des Aujeszky Virus gerichtet (Zuckermann et al., 1990, J. Virol. 64, 802-812).

Übertragen auf das System des BHV-1 bedeutet dies, daß das Fehlen des von der Funktion vergleichbaren Protein gIII in BHV-1 eine Reduktion der Immunogenität bewirken müßte. Um BHV-1 Impfstoffe mit hoher Immunogenität herzustellen, müssen daher BHV-1-Mutanten, die als Impfviren eingesetzt werden, Deletionen oder andere Variationen in Proteinen oder Protein-Abschnitten haben, die nicht Ziel relevanter Abwehrmechanismen sind.

Die vorliegende Erfindung betrifft nun:
1. Verwendung von BHV-1-Stämmen zur Herstellung von Impfstoffen gegen BHV-1 Infektionen die eine Unterscheidung geimpfter Tiere von feldinfizierten Tieren, erlauben, wobei diese BHV-1-Stämme dadurch gekennzeichnet sind, daß sie
   - in ihrem Genom Änderungen im Bereich enthalten, der für Glycoprotein g-IV kodiert,
   - diese Änderungen in Bereichen liegen, die für Glycoprotein g-IV nichtessentiell sind,
   - diese für Glycoprotein g-IV nichtessentiellen Bereiche Epitope betreffen, die im Glycoprotein g-IV des BHV Stammes Reg. Nr. I 1204 (Institut Pasteur vom 13.4.1992) dem Bereich der Aminosäure-Positionen 310-338 entsprechen,
   - in diesen für Glycoprotein g-IV des BHV Stammes Reg. Nr. I 1204 (Institut Pasteur vom 13.4.1992) nichtessentiellen Bereichen im Vergleich zu Feldviren ein oder mehrere Nukleotid(e) ausgetauscht, deletiert oder inseriert sind.
2. Verwendung gemäß Anspruch 1, wobei als BHV-1 Stamm der Stamm Reg. Nr. I 1204 (Institut Pasteur vom 13.4.1992) eingesetzt wird.
3. DNA-Sequenz die für das essentielle Glycoprotein g-IV des BHV-1-Stammes Reg. Nr. I 1204 (Institut Pasteur vom 13.4.1992) kodiert.
4. Protein g-IV des BHV-1-Stammes Reg. Nr. I 1204 (Institut Pasteur vom 13.4.1992).
5. Verwendung von Peptiden die homolog zu Aminosäure-Sequenzen von nichtessentiellen Bereichen in Glycoprotein g-IV in BHV-1 Feldviren sind und die heterolog zu den geänderten Aminosäure-Sequenzen Position 310-338 im Glycoprotein g-IV des BHV-1 Stammes Reg. Nr. I 1204 (Institut Pasteur vom 13.4.1992) sind in serologischen Verfahren zur Unterscheidung von Rindern, die mit BHV-1 Feldviren infiziert sind von Rindern, die mit Impfstoffen auf Basis des BHV-1 Stammes Reg. Nr. I 1204 (Institut Pasteur vom 13.4.1992) geimpft wurden.

Die vorstehend aufgeführten Begriffe haben folgende Bedeutung:
BHV bedeutet Bovines Herpesvirus
BHV-1
   bedeutet Bovines Herpesvirus vom Serotyp 1 nach der Nomenklatur in Virus Diseases in Laboratory and Captive Animals" F.J. Conraths, H. Ludwig, G. Darai 1988 Verlag Martinus Nijhoff, Boston.
BHV-1.3
   bedeutet Bovines Herpesvirus vom Subtyp 3 des Serotyps 1. Vertreter dieses Subtyps wurden erstmals isoliert in Australien in 1962 als Stamm N569 (French, 1962, Australian Veterinary Journal 38, 216ff) und als Stamm A663 in Argentinien (Carrillo et al. 1983, Zbl.Vet.Med.B 30, 327ff) bei Ausbrüchen von virusbedingten Encephalitiden in Rindern. Sowohl Metzler und Mitarbeiter (1986, Archives of Virology 87, 205ff) als auch Engels und Mitarbeiter (1986/87, Virus Research 6, 57ff) beschrieben diese Isolate, deren DNA-Restriktionsenzym-Fragment-Muster einheitlich und im Vergleich zu anderen Vertretern des BHV-Typs-1 zu unterscheiden war, als Subtyp 3 des Serotyps 1 von BHV. Auch der von Bartha und Mitarbeitern in Ungarn aus einem an Encephalitis erkrankten Rind isolierte Stamm Na67 (Bartha et al., 1969, Acta Veterinariae Academiae Scientarium Hungaricae 19, 145ff) zeigte nach Untersuchungen von Magyar und Mitarbeitern (1989, Acta Veterinaria Hungaricae 37, 3ff) das typische BHV-1.3-DNA-Muster. Studdert bezeichnet diese Virusgruppe mit "Bovine Encephalitis Herpesvirus (BEHV)" (Studdert, 1989, Vet.Rec. , 584).
   Die Vertreter des Subtyps 3 spielen quantitativ eine vollkommen untergeordnete Rolle im Vergleich zum Vorkommen der beiden übrigen Subtypen 1 und 2 des BHV-1 (BHV-1.1 und BHV-1.2). Die Subtypen 1.1 und 1.2 unterscheiden sich durch ein für sie jeweils charakteristisches DNA-Muster nach Behandlung ihrer genomischen DNA mit bestimmten Restriktionsenzymen (Engels et al. (1981), Arch. Virol. 67, 169 ff). Als typische Vertreter seien genannt der Stamm Cooper für BHV-1.1 und der Stamm Schönböken für BHV-1.2.
Feldviren
   sind Bovine Herpesviren vom Typ 1, die unter natürlichen Bedingungen vorkommen. Ihre DNA-Sequenzen kodieren für mindestens ein Epitop in nicht-essentiellen Teilen von essentiellen Proteinen, das in der überwiegenden Mehrzahl aller unter natürlichen Bedingungen vorkommenden Varianten gleich ist und damit als charakteristisch für BHV-1 Feldviren zu bezeichnen ist. Die Bildung von Antikörpern in Rindern gegen diese(s) Epitop(e) läßt daher auf eine Infektion mit BHV-1 Feldviren schließen.
Essentielles Protein
   bedeutet ein oder mehrere Proteine, die für die Virusvermehrung von BHV-1 in der Zellkultur notwendig sind. Diese Proteine werden vom Genom des BHV-1 kodiert und können Bestandteil des reifen Viruspartikels sein oder können zu seiner Vermehrung beitragen ohne Bestandteil des reifen Viruspartikels zu werden. Als Beispiele für essentielle Proteine des BHV-1 seien genannt gB und gD (benannt nach der Nomenklatur für HSV) (Wyler et al., Infectious Bovine Rhinotracheitis/Vulvovaginitis, in "Developments in Veterinary Virology", Vol. "Herpesvirus Diseases of Cattle, Horses, and Pigs", ed. by Wittmann, Kluwer Academic Publishers, 1989).
Nicht-essentielle Bereiche von essentiellen Proteinen
   bezeichnen Teile von essentiellen Proteinen. Veränderungen der Aminosäure-Sequenz in diesen Teilen sind ohne Blockierung der Funktion des Gesamtproteins für die Virusvermehrung möglich. Im Zusammenhang mit der vorliegenden Erfindung ist wichtig, daß diese nicht-essentiellen Bereiche auf Basis ihrer Aminosäure-Sequenz Epitope bilden können, die in BHV-1-infizierten Rindern zur Bildung von spezifischen Antikörpern führen.
   Geänderte Aminosäure-Sequenz bedeutet, daß die Aminosäure-Sequenz eines essentiellen Proteins eines BHV-1 Stammes in mindestens einem nicht-essentiellen Bereich im Vergleich zum Feldvirus so unterschiedlich ist, daß das im entsprechenden Protein des Feldvirus in diesem(n) Bereich(en) vorhandene Epitop nicht mehr im Protein des Virus mit der veränderten Aminosäure-Sequenz vorhanden ist. Rindern, die mit dem Virus, das die geänderte Aminosäure-Sequenz enthält, infiziert werden oder dieses Virus in inaktivierter Form oder Teile desselben appliziert bekommen, fehlen somit spezifische Antikörper gegen mindestens ein Epitop im Feldvirus, die sie nach einer Infektion mit einem Feldvirus bilden.
   Die geänderten Aminosäure-Sequenzen können in natürlich vorkommenden BHV-1 Stämmen auftreten, wie z.B. die Positionen 310 bis 338 im essentiellen Strukturprotein gIV (gD) des BHV-1 Stammes Reg. Nr. I 1204 (Institut Pasteur vom 13.4.1992); sie können bei Zellpassagen von Feldviren entstehen oder nach der Identifizierung von nicht-essentiellen Bereichen von essentiellen Proteinen durch Veränderung der DNA-Sequenz im Genom von BHV-1 über gentechnologische Methoden hergestellt werden.
   Die Änderung in der Aminosäure-Sequenz eines nicht-essentiellen Bereiches in einem essentiellen Protein kann auf einem Austausch einer oder mehrerer Aminosäure, Fehlen von einer oder mehreren Aminosäuren, der Inserierung von einer oder mehreren Aminosäuren oder aus jeden Kombination der genannten Möglichkeiten aufgrund von einer im Vergleich zum Feldvirus geänderten DNA-Sequenz des Virus-Genoms beruhen. Entscheidend ist, daß die Veränderung nicht die Funktion des essentiellen Proteins bei der Virusvermehrung blockiert und den Verlust mindestens eines Epitops zur Folge hat, wobei durchaus ein oder mehrere neue Epitope dabei entstehen können.
Geänderte DNA-Sequenz
   bedeutet, daß das Virusgenom eines BHV-1-Stammes im Vergleich zu Feldviren eine unterschiedliche Nukleotid-Sequenz als Folge eines oder mehrerer Nukleotid-Austausche, einer Deletion von einem oder mehreren Nukleotiden, einer Inserierung eines oder mehrerer Nukleotide oder jeder beliebigen Kombination dieser Möglichkeiten trägt. Die Änderung führt in jedem Fall in mindestens einem nicht-essentiellen Bereich eines essentiellen Virusproteins zum Verlust mindestens eines Epitops, wobei durchaus ein oder mehrere neue Epitope dabei entstehen können, ohne daß hierbei die Funktion des betroffenen Virusproteins für die Virusvermehrung blockiert sein darf.
Glykoprotein gIV
   bezeichnet das glykosylierte Strukturprotein des BHV-1, das als Homologon des gD von HSV bezeichnet wird (Wyler et al.; Developments in Veterinary Virology; Vol. Herpesvirus Diseases of Cattle, Horses, and Pigs; ed. Wittmann; Fluwer Academic Publishers, 1989). Die Nukleotid-Sequenz des Gens für gIV und seine Lokalisation auf dem Genom sind für die BHV-1 Stämme Schönböken und Australia (Beninga, Diplomarbeit der Fakultät für Biologie der Universität Tübingen, 1989) beschrieben. Das reife, ins extrazelluläre Viruspartikel eingebaute Glycoprotein gIV besitzt bei den typischen Vertretern der BHV Typen 1.1 und 1.2 ein in der SDS-Polyacrylamidgel-Elektrophorese ermitteltes, relatives Molekulargewicht von ca. 71Kda (Wyler et al., Infectious Bovine Rhinotracheitis/Vulvovaginitis, in "Developments in Veterinary Virology", Vol. "Herpesvirus Diseases of Cattle, Horses, and Pigs", ed. by Wittmann, Kluwer Academic Publishers, 1989). Das Glykoprotein gIV der BHV-1.3 Stämme besitzt ein relatives Molekulargewicht von ca. 68 KDa.
Bereich, der unmittelbar aus der Virushülle des Virus herausragt
   bedeutet die Region im Glykoprotein gIV, die - in der Orientierung des Proteins vom Aminoterminus in Richtung Carboxyterminus - kurz vor der sogenannten Membrandomäne liegt. Beninga (Diplomarbeit der Fakultat für Biologie der Universität Tübingen, 1989) konnte über die Sequenzierung des Gens für gIV der BHV-1 Stämme Schönböken und Australia die Aminosäure-Sequenz und daraus die Sekundärstruktur sowie die hydrophilen und hydrophoben Bereiche dieses Proteins bestimmen. Am Aminoterminus ist nach diesen Untersuchungen eine stark hydrophobe Region zu finden, an die sich eine Region von ca. 300 Aminsäuren anschließt, die hydrophile und hydrophobe Bereiche aufweist und relativ stark gefaltet ist. Es folgt in Richtung Carboxyterminus ein linearer Bereich mit vorwiegend hydrophilem Charakter. Dieser Bereich mit ungefähr 50 Aminosäuren beim BHV-1 Stamm Schönböken ragt unmittelbar aus der Membranhülle des Virus heraus und trägt die möglichen Änderungen in der Aminosäure-Sequenz gemäß 3 (oben). Unmittelbar in Richtung Carboxyterminus folgt hierauf eine sehr stark hydrophobe Region, die als Membrandomäne bezeichnet wird und das Glykoprotein in der Membran verankert.
Natürlicher Wirt
   bedeutet jedes Tier, das mit BHV-1 infiziert werden kann und dieses Virus vermehrt. Natürliche Wirte können sein Rind, Schwein und Ziege (Rolle und Mayr, Mikrobiologie, Infektions- und Seuchenlehre, F. Enke Verlag Stuttgart).
In Zellen passagierter BHV-1 Stamm
   bedeutet, daß ein BHV-1 Stamm in eukaryontischen Gewebekulturzellen vermehrt wird. Hierbei wird die Ernte einer Vermehrung auf Gewebekulturzellen oder ein BHV-1 Isolat aus einem Tier, eventuell nach Lagerung, auf Gewebekulturzellen inokuliert und nach der Virusvermehrung wieder geerntet. Die Anzahl der Vermehrungen auf den Gewebekulturzellen ist dabei abhängig vom Ziel der Gewebekulturpassagen. Soll das Virus zur Gewinnung größerer Mengen für analytische oder präparative Ansätze vermehrt werden, genügt eine kleine Zahl, z.B. 1 bis 10 Vermehrungszyklen. Soll das Virus durch die Gewebekulturpassagen in seinen biologischen oder biochemischen Eigenschaften, z.B. Virulenz, Aminosäure-Sequenz und/oder DNA-Sequenz, geändert werden, ist eine höhere Anzahl von Zellpassagen zu empfehlen. So konnte z.B, der ursprünglich für Rinder pathogene BHV-1 Stamm Schönböken durch ca. 200 Zellpassagen bis zur Apathogenität verändert werden.
   Durch die Auswahl der für die Zellpassagen eingesetzten Zellen kann das Auftreten von Veränderungen der biologischen oder biochemischen Eigenschaften beeinflußt werden. Zellen, die z.B. von nicht-natürlichen Wirten des BHV-1 abstammen, wie z.B. Hunde-Nieren-Zellen, können Selektionsvorteile für z.B. apathogene Mutanten des BHV-1 bieten, was zur Attenuierung eines ursprünglich pathogenen BHV-1 Stammes führen kann.
Bestimmung der DNA-Sequenz
   bedeutet, daß der zu untersuchende Virusstamm vermehrt, das Virusgenom isoliert und zumindest Teile hiervon in Vektoren molekular kloniert werden. Die Klone mit viralen Genen für essentielle Proteine können über DNA-Sonden durch Hybridisierung identifiziert werden. Als DNA-Sonden können z.B. DNA-Fragmente oder synthetische Oligonukleotide mit Nukleotid-Sequenzen von Genen für essentielle Proteine von BHV-1 oder anderer Herpesviren eingesetzt werden. Nukleotid-Sequenzen von BHV-1 Genen beziehungsweise DNA-Fragmente mit Teilen von oder mit gesamten BHV-1 Genen für essentielle Proteine des BHV-1, z.B. gD und gB, die als DNA-Sonden eingesetzt werden können, sind bekannt von Beninga (Diplomarbeit der Fakultät für Biologie der Univerßität Tübingen, 1989) und Chase und Mitarbeiter (Journal of Tissue Culture Methods, 11, 75ff, 1988, und J.Gen.Virol. 70, 1561ff, 1989). Mit Hilfe der identifizierten DNA-Klone, die DNA-Sequenzen des Virus-Genoms für nicht essentielle Proteine tragen, kann nach den an sich bekannten Methoden die Nukleotid-Sequenz dieser Virus-Gene bestimmt werden.
Direkt die Aminosäure-Sequenz bestimmen
   bedeutet, daß durch die an sich bekannten biochemischen Methoden die Aminosäure-Sequenz eines oder mehrerer essentiellen BHV-1 Proteine bestimmt wird. Vorher werden die essentiellen Proteine durch z.B. Immunaffinitätschromatographie unter Einsatz bekannter monoklonaler Antikörper gegen die essentiellen Virusproteine, wie z.B. gB und gD (Chase und Mitarbeiter, Journal of Tissue Culture Methods, 11, 75ff, 1988; Van Drunen Littel-van den Hurk und Mitarbeiter, Vaccine 8, 358ff, 1990; Fitzpatrick und Mitarbeiter, Virology 176, 145ff, 1990; Duque und Mitarbeiter, Vaccine 7, 513ff, 1989; Marshall und Mitarbeiter, Virology 165, 338ff, 1988), aus BHV-1 infizierten Zellen oder aus Viruspartikeln gereinigt. Alternativ können die zu sequenzierenden Proteine auch in eu- oder prokaryontischen Expressionssystemen synthetisiert und dann über Immunaffinitätschromatographie gereinigt werden.
Vergleich der Aminosäure-Sequenz
   bedeutet, daß die Aminosäure-Sequenz von essentiellen Proteinen des untersuchten BHV-1 Stammes mit der Aminosäure-Sequenz von den entsprechenden essentiellen Proteinen eines oder mehrerer BHV-1 Feldviren verglichen wird. Hierzu werden die zu vergleichenden Aminosäure-Sequenzen in gleicher Orientierung vom Aminoterminus zum Carboxyterminus schematisch so aneinandergelegt, daß an möglichst vielen Positionen identische Aminosäuren in den zu vergleichenden Sequenzen vorliegen. Um dies zu erreichen, können die einzelnen Aminosäure-Sequenzen durchaus auch schematisch getrennt und unterbrochen werden.
   Es werden Epitope in den essentiellen Proteinen von BHV-1 Feldviren gesucht, die in den essentiellen Proteinen des untersuchten BHV-1 Stammes nicht vorkommen. Regionen von mindestens 5 aufeinanderfolgenden Aminosäuren in der Aminosäure-Sequenz der essentiellen Proteine des Feldvirus (oder der Feldviren), die in den entsprechenden Proteinen des untersuchten BHV-1 Stamm nicht vorkommen, weisen z.B. auf mögliche Änderungen von Epitopen in dem untersuchten BHV-1 Stamm hin.
Spezifische Antikörper
   bezeichnen Seren oder Serumfraktionen von Tieren mit Antikörpern gegen Teile von essentiellen Proteinen von BHV-1 Feldviren, oder monoklonale Antikörper (in Form von Hybridoma-Kultur-Medien oder Ascites von mit Hybridomazellen behandelten Mäusen) gegen einzelne Epitope von essentiellen Proteinen von BHV-1 Feldviren. BHV-1 Stämme, die eine geänderte Aminosäure-Sequenz in den entsprechenden Bereichen ihrer essentiellen Proteine haben, den diese Antikörper in BHV-1 Feldviren erkennen, werden durch diese Antikörper nicht erkannt.
   Bevorzugt werden Seren oder Serum-Fraktionen von Rindern.
   Zur Herstellung dieser Antikörper werden Tiere, z.B. Rinder, die keine Antikörper gegen BHV-1 besitzen, mit Teilen von essentiellen Proteinen von BHV-1 Feldviren, gegebenenfalls unter Zusatz von Adjuvantien, durch ein- oder mehrmalige Applikation immunisiert. Die Seren der immunisierten Tiere können ungereinigt oder als Serumfraktionen die nur gegen die zur Immunisierung eingesetzten Antigene gerichtete Antikörper enthalten eingesetzt werden. Solche Serumfraktionen werden, z.B. durch Reinigung über Affinitätschromatographie erhalten.
   Die Rinder können auch mit intakten BHV-1 Feldviren immunisiert werden. Dann müssen die Serum-Fraktionen dieser Tiere, die Antikörper nur gegen einzelne Teile von essentiellen Proteinen von BHV-1 Feldviren enthalten, auf jeden Fall gereinigt werden. Dies kann dadurch erfolgen, daß die Seren mit gereinigten Teilen von essentiellen Proteinen von BHV-1 Feldviren als Antigen zur Adsorption von entsprechenden Antikörpern behandelt werden. Die adsorbierten Antikörper werden dann eluiert und gesammelt (Immun-Affinitätschromatographie).
   Ganz besonders bevorzugt werden monoklonale Antikörper aus z.B. Mäusen, die mit intakten BHV-1 Feldviren oder gereinigten essentiellen Proteinen von BHV-1 Feldviren oder mit Teilen von essentiellen Proteinen von BHV-1 Feldviren immunisiert wurden. Die Immunisierung der Tiere, Fusion ihrer Milzzellen mit Myelomzellen und Gewinnung der Antikörper geschieht gemäß den für die Herstellung von monoklonalen Antikörpern an sich bekannten Methoden ("Antibodies, a Laboratory Manual"; E.Harlow und D.Lane; Cold Spring Harbor Laboratory; 1988). Wurden die Mäuse mit intakten BHV-1 Feldviren immunisiert, müssen die Hybridomakulturen selektioniert werden, die Antikörper gegen essentielle Proteine von BHV-1 Feldviren bilden. Die Selektion dieser Hybridoma-Kulturen kann z.B. durch Prüfung ihrer Zellkultur-Überstände in einem ELISA mit essentiellen Proteinen von BHV-1 Feldviren oder Teilen dieser Proteine als Antigen erfolgen.
Immunologische Methoden
   bedeuten Techniken, mit deren Hilfe unter Einsatz von Seren, Serum-Fraktionen oder monoklonalen Antikörpern Teile von essentiellen Proteinen von BHV-1 untersucht werden können. (Zur Übersicht: "Antibodies, a Laboratory Manual"; E.Harlow und D.Lane; Cold Spring Harbor Laboratory; 1988). Die hier verwendeten Seren, Serum-Fraktionen oder monoklonalen Antikörper erkennen Teile von essentiellen Proteinen von BHV-1 Feldviren. Es werden BHV-1 Stämme gesucht, deren essentielle Proteine nicht von diesen Seren, Serum-Fraktionen oder monoklonalen Antikörpern erkannt werden, die in essentiellen Proteinen im Vergleich zu Feldviren somit verschiedene Epitope besitzen. Die Wahl der geeigneten Technik kann sich nach dem zur Verfügung stehenden Material an Serum, Serum-Fraktion oder monoklonalem Antikörper richten.
   Als Beispiel einer Technik sei der Immunoblot (Western-Blot) genannt. Hierbei werden die Proteine von BHV-1 in der an sich bekannten Methode der SDS-Polyacrylamidgelelektrophorese aufgetrennt und die Proteine anschließend im elektrischen Feld auf Filterpapier, z.B. Nitrozellulose-Filter, übertragen. Dieses Filter wird dann mit Seren oder Serum-Fraktionen mit Antikörpern gegen Teile von essentiellen Proteinen von BHV-1 Feldviren oder mit monoklonalen Antikörpern gegen essentielle Proteine von BHV-1 Feldviren inkubiert. Die Bindung dieser Antikörper an Antigene auf dem Filter wird durch an sich bekannte enzymvermittelte Farbreaktionen visualisiert ("Antibodies, a Laboratory Manual"; E.Harlow und D.Lane; Cold Spring Harbor Laboratory; 1988).
   Ein weiteres Beispiel ist der an sich bekannte Enzyme-Linked-Immunosorbent-Assay, der ebenfalls die Bindung von Antikörpern an Antigene über eine enzymgekoppelte Farbreaktion visualisiert, bei dem BHV-1 als Antigen eingesetzt wird ("Antibodies, a Laboratory Manual"; E.Harlow und D.Lane; Cold Spring Harbor Laboratory; 1988).
   Ein weiteres Beispiel ist der an sich bekannte Immunfluoreszenstest, der eine Antikörper-Bindung an ein Antigen über Fluoreszenz nachweist.
Prüfung auf Antigenität
   bedeutet, daß die entsprechende Aminosäure-Sequenz in BHV-1 Feldviren, die in dem untersuchten BHV-1 Stamm nicht identifiziert werden konnte, auf ihre Fähigkeit untersucht wird, im Tier als Antigen zu wirken, d.h. die Bildung von Antikörpern zu induzieren. Dies kann dadurch erfolgen, daß z.B.
   - diese Aminosäure-Sequenz durch Beurteilung ihrer Wasserlöslichkeit und der Voraussage von möglichen Sekundärstrukturen auf die Wahrscheinlichkeit einer Oberflächen-Exposition dieser Sequenz im essentiellen Protein von BHV-1 Feldviren untersucht wird.
   - ein synthetisch hergestelltes Peptid mit der Aminosäure-Sequenz der BHV-1 Feldviren, die im untersuchten BHV-1 Stamm nicht identifiziert werden konnte, in Tiere, bevorzugt in Rinder appliziert wird. Nach dieser Immunisierung werden z.B. in wöchentlichen Intervallen Blutproben der immuniserten Tiere entnommen und die Seren dieser Tiere auf den Gehalt an Antikörpern gegen dieses Peptid untersucht.
   - die Seren von Tieren, bevorzugt von Rindern, die mit BHV-1 Feldviren infiziert wurden, auf den Gehalt an Antikörpern gegen die Aminosäure-Sequenz in essentiellen Proteinen von BHV-1 Feldviren, die im untersuchten BHV-1 Stamm nicht identifiziert werden konnte, untersucht werden. Hierzu werden z.B. die Seren auf ihre Fähigkeit untersucht, ein synthetisch hergestelltes Peptid mit der Aminosäure-Sequenz von BHV-1 Feldviren, die im untersuchten BHV-1 Stamm nicht identifiziert werden konnte, zu binden. Als Technik seien der an sich bekannte Enzyme-Linked-Immunoadsorbent-Assay (ELISA) oder der an sich bekannte Immuno-Dot-Blot mit synthetischen Peptiden als Antigen genannt.
Epitop
   ist eine spezifische Bindungsstelle für Antikörper auf Basis einer Aminosäure-Sequenz oder gegebenenfalls einer glykosylierten Aminosäure-Sequenz.
Nukleotid Sequenzen
   sind Teile der genomischen DNA von BHV-1.
Veränderungen von Nukleotid-Sequenzen
   umfassen alle Methoden, die geeignet sind, die Nukleotid-Sequenzen, die für essentielle Proteine von BHV-1 kodieren, zu ändern. Die Änderung kann beruhen auf
   - Deletion von einem oder mehreren Nukleotiden und/oder
   - Insertion von einem oder mehreren Nukleotiden und/oder
   - Austausch von einem oder mehreren Nukleotiden.
   Eine Auswahl von Methoden zur Änderung von Nukleotid-Sequenzen ist gegeben in "Molecular Cloning" 2nd edition, 1989, ed. J.Sambrook, E.F.Fritsch, T. Maniatis, Cold Spring Harbor Laboratory Press. Die Änderung der Nukleotid-Sequenz soll zur Eliminierung von Epitopen in essentiellen Proteinen von BHV-1 führen, ohne daß hierbei die biologische Funktion dieses Proteins für die Virusvermehrung blockiert wird.
Deletion
   heißt, daß aus einer Sequenz ein oder mehrere Bausteine ohne Ersatz entfernt werden, ohne daß die Funktion des Proteins blockiert wird.
Substitution
   heißt, daß ein Baustein aus einer Sequenz deletiert wird und durch einen anderen Baustein an der gleichen Stelle ersetzt wird, ohne daß die Funktion des Proteins blockiert wird.
Isolierung der DNA
   heißt, daß über an sich bekannte Methoden der Molekulargenetik die DNA aus einem DNA-haltigen Gemisch (z.B. aus gereinigten Viruspartikeln von BHV-1) extrahiert wird (Virologische Arbeitsmethoden Bd. III, ed. A.Mayr; P.A.Bachmann; B.Mayr-Bibrack; G.Wittmann; Gustav Fischer Verlag).
   Genom molekular kloniert (vgl. Punkt 8 oben) bedeutet, daß die genomische DNA von BHV-1 isoliert wird und die DNA-Fragmente, die Gene oder DNA-Sequenzen von Genen für essentielle Proteine von BHV-1 enthalten in gängige DNA-Vektoren (z.B. bakterielle Plasmide wie pBR322, pUC18/19 u.a.) inseriert werden. Die Identifizierung von DNA-Fragmenten aus dem Genom von BHV-1, die Gene oder Teile von Genen für essentielle Proteine enthalten kann erfolgen
   - dadurch, daß die DNA-Sequenzen für Gene von essentiellen Proteinen von BHV-1 bekannt sind (z.B. gI beschrieben in Chase et al., 1989, J.gen.Virol. 70, 1561-1569) oder
   - über Homologien von Genen für essentielle Proteine von anderen Herpesviren, wie z.B. Herpes Simplex Virus, Aujeszky Virus, Equines Herpesvirus 1 und 4, für die die DNA-Sequenzen ihrer Gene für essentielle Proteine bekannt sind. Über DNA/DNA-Hybridisierungen dieser DNA-Sequenzen mit genomischen DNA-Fragmenten von BHV-1 können dessen DNA-Fragmente mit Genen für essentielle Proteine gefunden werden.
   Eine Auswahl an Methoden zur Herstellung und Klonierung von DNA-Fragmente gibt "Molecular Cloning" 2nd edition, 1989, ed. J. Sambrook, E.F. Fritsch, T. Maniatis, Cold Spring Harbor Laboratory Press. Diese Vektoren mit den BHV-1 DNA-Fragmenten als Inserts dienen z.B. der Herstellung von identischen Kopien der ursprünglich isolierten DNA-Fragmente mit Genen oder DNA-Sequenzen von Genen für essentielle Proteine von BHV-1.
Als Vektoren,
   die zur Klonierung von DNA-Fragmenten des Genoms von BHV-1 und/oder gegebenenfalls zur gezielten Veränderung von DNA-Sequenzen in Genen für essentielle Proteine von BHV-1 eingesetzt werden können, seien genannt alle gängigen bakteriellen oder eukaryontischen Plasmid-Vektoren (wie z.B. pBR322, puC18/19 u.a.), bakterielle Phagen (wie z.B. Lambda, M13 u.a.). Möglichkeiten zur Inserierung der BHV-1 DNA-Fragmente in Vektoren, deren Vermehrung in Wirtszellen und die Reisolierung der DNA-Inserts sind im Detail in "Molecular Cloning" 2nd edition, 1989, ed. J.Sambrook, E.F.Fritsch, T.Maniatis, Cold Spring Harbor Laboratory Press beschrieben.

Verwendung von essentiellen Proteinen von BHV-1 Stammen mit Änderungen in nicht-essentiellen Bereichen zur
Identifizierung von BHV-1 Stämmen
bedeutet, daß ein BHV-1 Stamm mit geänderten Aminosäure-Sequenzen in essentiellen Proteinen, dessen Fähigkeit zum Einsatz gemäß (1) belegt wurde, zur Identifizierung weiterer gemäß (1) nutzbarer BHV-1 Stämme dient.
a. Die Identifizierung von geänderten Aminosäure-Sequenzen lokalisiert auch nicht-essentielle Regionen in essentiellen Proteinen von BHV-1, deren Aminosäure-Sequenz ohne Blockierung der biologischen Funktion dieser Proteine für die Virusvermehrung geändert werden können. Das vereinfacht die Suche nach anderen BHV-1 Stämmen mit geänderten Aminosäure-Sequenzen in essentiellen Proteinen, da die zu untersuchenden Regionen ihrer essentiellen Proteine enger eingegrenzt werden können.
b. Die Kenntnis von nicht-essentiellen Regionen in essentiellen Proteinen von BHV-1 vereinfacht die Herstellung von BHV-1 Stämmen gemäß (4), da der Bereich in essentiellen Proteinen hiermit bekannt ist, der über Änderungen der DNA-Sequenz im Genom von BHV-1 geändert werden kann.
c. Antikörper, hergestellt gegen geänderte Aminosäure-Sequenzen in nicht-essentiellen Teilen von essentiellen Proteinen von BHV-1 Stämmen können zur Identifizierung weiterer BHV-1 Stämme genutzt werden, die identische Änderungen tragen.
d. Die essentiellen Proteine von BHV-1, die geänderte Aminosäure-Sequenzen tragen, können als immunogene Bestandteile in BHV-1 Impfstoffen eingesetzt werden. Hierzu müssen diese Proteine in reiner Form hergestellt werden. Dies kahn z.B. durch bekannte biochemische oder immunologische Reinigungs-Verfahren aus Virus-haltigem Material oder durch Expression von Genen, die für diese Proteine kodieren, in bakteriellen oder eukaryontischen Expressionssystemen erfolgen. Nach Verifizierung der Immunogenität dieser Proteine können diese gegebenenfalls unter Zusatz von Adjuvantien als Impfstoffe zur Bekämpfung von BHV-1 Infektionen eingesetzt werden.

Isolierung der Proteine zur Herstellung von Proteinen gemäß (10) umfaßt die bekannten Methoden zur Isolierung von Virusproteinen aus Virus-haltigem Material. Als Beispiele seien genannt:
a. die Reinigung von Proteinen durch Antikörper, die spezifisch für das zu reinigende Protein sind in z.B. der Immun-Affinitätschromatographie ("Antibodies, a Laboratory Manual"; E. Harlow und D. Lane, Cold Spring Harbor Laboratory; 1988).
b. die Reinigung von Virusproteinen über chromatographische Methoden, wie z.B. Ionenaustausch-Chromatographien auf Basis ihrer spezifischen Ladungsverhältnisse oder Molekular-Sieb-Chromatographien auf Basis ihrer Molekulargewichte.

Expression von Proteinen zur Herstellung gemäß (10) in pro- oder eukaryontischen Expressionssystemen umschreibt die bekannten Methoden der Molekulargenetik zur Herstellung von Proteinen mit Hilfe klopierter Gene. Eine Übersicht über die gängigen Expressionssysteme und Anleitungen für den Umgang mit diesen ist gegeben in "Molecular Cloning" 2nd edition, 1989, ed. J. Sambrook, E.F. Fritsch, T. Maniatis, Cold Spring Harbor Laboratory Press. Hierfür müssen die DNA-Fragmente aus dem Genom von BHV-1 isoliert werden, die Gene für die essentiellen Proteine enthalten. Diese DNA-Fragmente werden so in die gängigen Expressions-Vektoren inseriert, daß eine Synthese der kodierten Proteine möglich ist. Als Expressionssysteme seien genannt:
a. Prokaryontische Systeme wie z.B. das Lambda-gt-11 System für E. Coli,
b. eukaryontische Systeme wie z.B. Hefe-Expressionssysteme, virale Vektorsysteme, wie z.B. Vaccinia-Systeme oder Baculo-Virus-Systeme.

Serologische Verfahren zur Unterscheidung von Rindern, die mit BHV-1 Feldviren infiziert sind, von Rindern, die mit Impfstoffen gemäß (1) geimpft wurden und/oder nicht mit BHV-1 infiziert wurden, umfassen Untersuchungen an Seren von Rindern auf ihren Gehalt an Antikörpern gegen die Aminosäure-Sequenzen in essentiellen Proteinen von BHV-1 Feldviren, die in den zur Herstellung von Impfstoffen gemäß (1) eingesetzten BHV-1 Stämme geändert sind. Der Nachweis von Antikörpern in Seren gegen die Aminosäure-Sequenz, wie sie in BHV-1 Feldviren vorkommt, erlaubt die Interpretation, daß die Tiere , aus denen die Seren gewonnen wurden, mit einem BHV-1 Feldvirus infiziert sind oder wurden. Tiere, deren Seren diese Aminosäure-Sequenz aus BHV-1 Feldviren nicht erkennen, sind mölgicherweise mit einem BHV-1 Impfstoff gemäß (1) auf Basis eines BHV-1 Stammes mit einer geänderten Aminosäure-Sequenz immunisiert worden und sind auf jeden Fall nicht mit einem BHV-1 Feldvirus infiziert.

Als Methoden zur Bestimmung von Antikörpern gegen Aminosäure-Sequenzen in BHV-1 Feldviren sei ein ELISA genannt. Als Antigen wird in diesem ELISA ein kurzkettiges, z.B. chemisch synthetisiertes, Peptid mit der Aminosäure-Sequenz aus BHV-1 Feldviren, die in den BHV-1 Stämmen gemäß (1) geändert ist, eingesetzt.

Soll darüberhinaus untersucht werden, ob die gleichen Tiere, die keine Antikörper gegen die Aminosäure-Sequenz von BHV-1 Feldviren haben, mit Impfstoffen gemäß (1) immunisiert wurden, werden die Seren dieser Tiere auf weitere BHV-1 spezifische Antikörper, z.B. in einem ELISA mit kompletten BHV-1 Partikeln als Antigen, untersucht. Der Nachweis dieser Antikörper belegt in Kombination mit den oben genannten ELISA-Ergebnissen, daß die Spender-Tiere dieser Seren mit Impfstoffen gemäß (1) immunisiert wurden.

### A. Identifizierung von BHV-1-Stämmen mit geänderten Aminosäure-Sequenzen gemäß 9 (oben)

### Vermehrung des BHV-1-Stammes, Genom-Reinigung und molekulare Klonierung gemäß 9a-1

Für die Vermehrung zur Identifizierung sind alle BHV-1-Stämme geeignet. Bevorzugt werden Stämme des BHV-1 vom Subtyp 3 (BHV-1.3). Besonders bevorzugt ist der BHV-1.3-Stamm N569.

Der Stamm N569 wurde am 13.April 92 gemäß Budapester-Vertrag unter der Reg.Nr. I 1204 beim Institut Pasteur CNCM hinterlegt.

Die Vermehrung der Viren erfolgt in üblicher Weise in Gewebekulturen animaler Zellen als Primärzellen oder permanenten Zell-Linien, z.B. in Rinder-Zellen, Affen-Zellen, Schweine-Zellen oder Hunde-Zellen, bevorzugt in Rindernieren-Zellen wie z.B. der permanenten Rindernieren-Zelle MD BK (ATCC CCL22 oder deren Abkömmlingen) oder der primären Rindernieren-Zelle EBK oder Affennieren-Zellen wie der permanenten Affennieren-Zelle Vero (ATCC CRL1586, CRL1587 oder deren Abkömmlinge) oder in Schweinenieren-Zellen wie der permanenten Schweinenieren-Zelle PK15 (ATCC CCL33 oder deren Abkömmlinge) oder in Hundenieren-Zellen wie der permanenten Hundenieren-Zelle MDCK (ATCC CCL34 oder deren Abkömmlinge).

Die Vermehrung erfolgt in an sich bekannter Weise in stationären, Roller oder Carrier Kulturen in Form von geschlossenen Zellverbänden oder in Suspensions-Kulturen. Als Vermehrungsmedien für die Zellen werden eingesetzt alle an sich bekannten Zellkulturmedien z.B. beschrieben im Produktkatalog der Fa. Flow Laboratories GmbH, Post 1249, 5309 Meckenheim, wie insbesondere das Minimal Essential Medium (MEM), das als wesentliche Bestandteile Aminosäuren, Vitamine, Salze und Kohlenhydrate enthält, komplettiert mit Puffersubstanzen wie z.B. Natrium-Bicarbonat oder (Hydroxyethylpiperazin-N-2-ethansulfonsäure (Hepes) und gegebenenfalls Tierseren, wie z.B. Seren von Rindern, Pferden bzw. deren Foeten. Besonders bevorzugt wird der Einsatz von foetalem Kälberserum in einer Konzentration von 1-30 Vol-%, vorzugsweise 2-10 Vol-%.

Die zur Vermehrung der Viren dienenden Zellen und Zellrasen werden in üblicher Weise nahezu bis zur Konfluenz oder bis zu optimalen Zelldichte vermehrt. Vor ihrer Infektion mit Viren wird bevorzugt das Zellvermehrungsmedium entfernt und die Zellen bevorzugt mit Virusvermehrungsmedium gewaschen. Als Virusvermehrungsmedien werden eingesetzt, alle an sich bekannten Zellkulturmedien, wie insbesondere das oben genannte MEM. Danach wird mit einer Virussuspension infiziert. In der Virussuspension liegt das Virus im Virusvermehrungsmedium derart verdünnt vor, daß mit einer MOI (= multiplicity of infection entspricht infektiöse Viruspartikel auf vorhandene Zellen) von 0,01-50, bevorzugt 0,10-10 infiziert wird.

Die Vermehrung der Viren erfolgt mit oder ohne Zusatz von Tierseren. Für den Fall, daß Serum eingesetzt wird, wird dieses zum Vermehrungsmedium in einer Konzentration von 1-30 Vol.-%, vorzugsweise 2-10 Vol-% zugegeben.

Infektion und Virus-Vermehrung erfolgen bei Temperaturen zwischen Raumtemperatur und 40°C, bevorzugt zwischen 32 und 39°C, besonders bevorzugt bei 37°C über mehrere Tage, bevorzugt bis zur vollständigen Zerstörung der infizierten Zellen.

Das virushaltige Medium der infizierten Zellen wird weiter aufgearbeitet, z.B. durch Entfernung der Zelltrümmer mittels Filtration mit Porengrößen von z.B. 0,1-0,45 µm und/oder Zentrifugation bis zu 10.000 g.

Filtrat oder Zentrifugationsüberstand werden zur Virusanreicherung und -reinigung verwendet. Dazu werden Filtrat oder Überstand einer hochtourigen Zentrifugation bis zur Sedimentation der Viruspartikel unterworfen. Gegebenenfalls können weitere Reinigungsschritte durch z.B. Zentrifugation in einem Dichtegradienten angeschlossen werden.

Das Genom der wie oben beschrieben vermehrten und gereinigten Viren wird isoliert und gereinigt.

Die Extraktion von nativer viraler DNA erfolgt bevorzugt durch die Behandlung der gereinigten Virionen mit wäßrigen Lösungen von Detergentien und Proteasen.

Als Detergentien seien genannt anionische, kationische, amphotere, nicht-ionische Detergentien. Bevorzugt werden ionische Detergentien eingesetzt. Besonders bevorzugt werden Natriumdodecylsulfat, Natriumlaurylsulfat.

Als Proteasen seien genannt, alle Proteasen, die in Gegenwart von Detergens arbeiten, wie z.B. Proteinase K und Pronase. Bevorzugt sei genannt Proteinase K.

Detergentien werden in Konzentrationen von 0,1 - 10 Vol-% eingesetzt, bevorzugt sind 0,5-3 Vol-%.

Proteasen werden in Konzentrationen von 0,01 - 10 mg pro ml Viruslysat eingesetzt, bevorzugt sind 0,05 - 0,5 mg pro ml Viruslysat.

Es wird bevorzugt in wäßriger gepufferter Lösung in Gegenwart von DNase Inhibitoren gearbeitet. Als Puffersubstanzen seien genannt: Salze schwacher Säuren mit starken Basen wie z.B. Tris(hydroxymethylaminomethan), Salze starker Säuren mit schwachen Basen wie z.B. primäre Phosphate oder Gemische derselben.

Bevorzugt sei das folgende Puffersystem genannt: Tris(hydroxymethylaminomethan).

Die Puffersubstanzen oder Puffersysteme werden in Konzentrationen eingesetzt, die pH-Werte gewährleisten, bei denen die DNA nicht denaturiert. Bevorzugt sind pH-Werte von 5-9, besonders bevorzugt 6-8,5, ganz besonders bevorzugt 7-8, insbesondere sei Arbeiten im neutralen Bereich genannt.

DNase-Inhibitoren sind z.B. Ethylendiamintetraessigsäure in Konzentrationen von 0,1 - 10 mM, bevorzugt ist ca. 1 mM.

Anschließend werden die lipophilen Bestandteile des Viruslysats extrahiert. Als Extraktionsmittel dienen Lösungsmittel wie Phenol, Chloroform, Isoamylalkohol oder deren Gemische. Bevorzugt wird zunächst ein Gemisch aus Phenol und Chloroform/Isoamylalkohol eingesetzt, wobei die Extraktion in einer oder mehreren Stufen erfolgt.

In der letzten Stufe der Extraktion wird bevorzugt Chloroform/Isoamylalkohol eingesetzt. Alternativ kann zunächst Phenol und anschließend Chloroform/Isoamylalkohol eingesetzt werden.

Weitere Methoden zur Isolierung der Viren DNA sind z.B. Zentrifugation eines Viruslysats in einem CsCl -Dichtegradienten oder in der Gelelektrophorese (Sharp et al.Biochem. 1973 (12) S. 3055-3063).

Die Extraktion von Nukleinsäuren wird in "Molecular Cloning", A Laboratory Manual, 2nd Edition 1989, ed J. Sambrook, E.F. Fritsch und T. Maniatis, Cold Spring Harbor Laboratory Press beschrieben.

Die so extrahierte DNA wird bevorzugt aus der wäßrigen Lösung mit z.B. Alkohol, bevorzugt mit Ethanol oder Isopropanol und unter Zusatz von monovalenten Salzen wie z.B. Alkalichloride oder -acetate, bevorzugt Lithiumchlorid, Natriumchlorid oder Natriumacetat, Kaliumacetat, ausgefällt.

Die Konzentration an Alkohol liegt hierbei zwischen 40 und 100 Vol-%, bevorzugt zwischen 60 und 80 Vol%, besonders bevorzugt bei ca. 70 Vol-%.

Die Chlorid- oder Acetatkonzentration liegt zwischen 0,01 oder 1 molar, bevorzugt zwischen 0,1 und 0,8 molar, Wird LiCl eingesetzt, liegt seine Konzentration zwischen 0,1 und 1 molar, bevorzugt zwischen 0,4 und 0,8 molar.

Methoden zur Präzipitation von Nucleinsäuren sind in "Molecular Cloning" loc. cit. ausführlich beschrieben. Die präzipitierte DNA wird durch z.B. Zentrifugation aus der wäßrigen Suspension isoliert, vorzugsweise mit Alkohol, z.B. 70 Vol-% Ethanol, gewaschen und schließlich in wäßriger Pufferlösung resolubilisiert.

Als Puffersubstanz sei genannt Tris(hydroxymethyl)aminomethan in Konzentrationen von 1-100 mM, bevorzugt wird 10-50 mM. Bevorzugte pH-Werte sind 6-8,5, besonders bevorzugt 7-8.

Als weitere Zusätze seien genannt, z.B. EDTA (Ethylendiamintetraessigsäure) in Konzentration von 0,1 bis 10 mM, bevorzugt 1 bis 10 mM.

Alternativ kann die präzipitierte DNA auch in 0,1 x SSC-Puffer (Molecular Cloning, loc. cit.) oder in Ammoniumcarbonat-Puffer resolubilisiert werden.

Die so gereinigte virale DNA wird mit Restriktionsenzym nach Vorschrift der Hersteller behandelt. Geeignete Restriktionsenzyme sind solche, die mindestens eine für sie spezifische Schnittstelle auf dem Virusgenom erkennen.

Die hierbei enstandenen Genomfragmente werden nach ihrer z.B. elektrophoretischen Auftrennung aus dem Trenngel (z.B. Agarose) isoliert und das Fragment, das die zu untersuchende DNA-Sequenz enthält, in ein Plasmid oder einen Phagenvektor inseriert.

Als Methoden zur Auftrennung der DNA Fragmente kommen elektrophoretische und chromatographische Verfahren in Frage.

Bei den chromatographischen Verfahren sei genannt Gelfiltration.

Bei den elektrophoretischen Verfahren seien als Träger genannt Agarbse oder Polyacrylamid. Als Elektrophoresepuffer seien genannt z.B. Ethylendiamintetraessigsäure, Phosphat/Puffer (EPP) Tris(hydroxymethyl)aminomethan-borat-ethylendiamintetraessigsäure-Puffer (TBE) der wie folgt zusammengesetzt ist:
- Tris: 10-100 mM, bevorzugt 40-90 mM, besonders bevorzugt 80-90 mM,
- Borsäure: 10-100 mM, bevorzugt 40-90 mM, besonders bevorzugt 80-90 mM,
- EDTA: 1-10 mM, bevorzugt 1-2,5 mM
- pH: 7-9, bevorzugt 8-8,5
oder
Tris-(hydroxymethyl)aminomethan-Acetat-Ethylendiamintetraessigsäure-Puffer (TAE) der wie folgt zusammengesetzt ist:
- Tris: 10-100 mM, bevorzugt 30-90 mM, besonders bevorzugt 40 mM,
- Natrium-Acetat: 1-100 mM, bevorzugt 5-50 mM,
- EDTA: 1-10 mM, bevorzugt 1-2,5
- pH: 7-9, bevorzugt 7,5-8,5.

Eine detaillierte Aufstellung und Beschreibung von Elektrophoresepuffern ist in
- Current Protocols in Molecular Biology 1987-1988, Verlag Wiley-Interscience, 1987
- A Practical Guide to Molecular Cloning, B, Perbal, 2^{nd} edition Verlag Wiley-Interscience, 1988
- Molecular Cloning, loc. cit.
- Virologische Arbeitsmethoden, Band III Gustav Fischer Verlag, 1989
beschrieben.
Die Durchführung des Verfahrens ist beschrieben in "Molecular Cloning" loc cit, oder in Virolog. Arbeitsmethoden Band loc. cit.

Die Isolierung des zu untersuchenden DNA-Fragments erfolgt aus dem Träger z.B. durch Elektroelution des das Fragment enthaltenden Trägerbereichs. Alternativ durch low-melting-Agaroseverfahren (Molecular cloning loc. cit.) oder durch Adsorption des DNA-Fragments an Glasoberflächen (Gene-clean® Methode).

Zur Insertion des DNA-Fragments werden doppelsträngige Plasmid oder Phagenvektor DNA-Moleküle mit Restriktionsenzymen behandelt, so daß für die Inserierung geeignete Enden entstehen.

Als Plasmide dienen z.B. pAT153, pACYC184, pUC18/19, pBR322, pSP64/65.

Als Phagenvektoren dienen Lambdaphagenvarianten wie z.B. -ZAP, -gt10/11 oder Phage M13mp18/19.

Die einsetzbaren Restriktionsenzyme sind an sich bekannt z.B. aus Gene Bnd 92 (1989) Elsevier Science Publishers BV Amsterdam.

Das mit Restriktionsenzym behandelte Plasmid oder der Phagenvektor wird mit einem Überschuß des zu inserierenden DNA-Fragments z.B. etwa im Verhältnis 5 zu 1 gemisch und mit DNA-Ligasen behandelt um das DNA-Fragment End-zu-End in den Vektor kovalent zu binden.

Ligasen sind Enzyme, die in der Lage sind zwei DNA-Moleküle über 3'-OH-5'-Reste zu verbinden.

Der Ligierungsansatz wird zur Vermehrung der Plasmide oder Phagen in pro- oder eukaryontische Zellen, bevorzugt in Bakterien, eingebracht und diese vermehrt.

Als Bakterien dienen z.B. Escherichia coli Stamm K-12 und seine Derivate z.B. K 12-600 (Molecular cloning loc. cit.).

Die Vorbereitung des Ligierungsansatzes und der Bakterienkultur erfolgt in an sich bekannter Weise wie in Molecular Cloning loc. cit. beschrieben.

Die Bakterien, die Plasmide mit inserierter Fremd-DNA enthalten bzw. Phagen mit Fremd-DNA, werden selektiert.

### Bestimmungen der DNA-Sequenz von viralen Genen und Ableitung der aus der ermittelten Sequenz resultierenden Aminosäure-Sequenz der entsprechenden Proteine gemäß 8 a-1

Die Nukleotid-Sequenz der oben hergestellten, klonierten Virus-DNA-Fragmente bzw. Subklone dieser werden nach den gängigen Methoden, wie sie z.B. in Molecular Cloning, loc. cit.; A Practical Guide to Molecular Cloning, ed. B. Perbal, A Wiley-Interscience Publication oder Virologische Arbeitsmethoden Bd. III, ed. A. Mayr, P.A. Bachmann, B. Mayr-Bibrack und G. Wittmann, Gustav Fischer Verlag, beschrieben sind, bestimmt.

Aus den Nukleotid-Sequenzen werden dann über den an sich bekannten genetischen Kode die Aminosäure-Sequenzen der viralen Proteine bestimmt (Biochemie, A.L.Lehninger Verlag Chemie 1977).

### Direkte Aminosäure-Sequenzierung von viralen Proteinen gemäß 8 a-2

Alternativ zur Bestimmung der Aminosäure-Sequenz über DNA-Sequenzierung kann die Sequenzierung der Aminosäure-Bausteine eines Proteins auch direkt am gereinigten Protein erfolgen. Eine detaillierte Beschreibung verschiedener Methoden ist in Virologischen Arbeitsmethoden, Bd. III, ed. A. Mayr, P.A. Bachmann, B. Mayr-Bibrack und G. Wittmann, Gustav Fischer Verlag, beschrieben.

### Identifizierung von geänderten Aminosäure-Sequenzen in viralen Proteinen gemäß 8 b

Durch den Vergleich der Aminosäure-Sequenz des untersuchten BHV-1-Stammes mit der entsprechenden Aminosäure-Sequenz anderer BHV-1-Stämme und/oder -Isolate oder Feldviren werden veränderte Regionen in Proteinen identifiziert. Dabei wird eine veränderte Region als geeignet angesehen, wenn die Veränderung den Verlust mindestens eines Epitopes bewirkte, z.B. wenn die Aminosäure-Sequenz sich in mindestens 5 aufeinanderfolgenden Aminosäuren vom Vergleichsstamm unterscheidet. Dabei können durchaus ein oder mehrere neue Epitope entstanden sein.

### Immunologische Methode zur Identifizierung von BHV-1 Stämmen mit veränderten Regionen in Virusproteinen gemäß 8c

Durch poly- oder monoclonale Antikörper, die spezifisch nur Teile eines einzelnen Proteins von BHV-1 Feldviren erkennen, können alternativ zur DNA-Sequenzierung oder direkten Aminosäure-Sequenzierung veränderte Regionen in Virusproteinen identifiziert werden. Hierzu wird die Bindungsfähigkeit dieser Antikörper an den zu untersuchenden BHV-1-Stamm geprüft. Als Methoden seien hier beispielsweise genannt:
Immunfluoreszens-Test, ELISA, ImmunoBlot. Bindet der Antikörper im Gegensatz zu BHV-1-Feldviren nicht an den zu untersuchenden BHV-1-Stamm, so trägt der letztere eine veränderte Aminosäure-Sequenz in dem Teil des Proteins, für das der Antikörper spezifisch ist.

### B. Prüfung auf Antigenität gemäß 8 d

### B. 1. Theoretische Bewertung der Antigenität einer Aminosäure-Sequenz

Der physikochemische Charakter einer Aminosäure-Sequenz (z.B. das Ausmaß des hydrophilen oder hydrophoben Verhaltens) gibt Hinweise auf eine mögliche Antigenität dieser Aminosäuresequenz. Über gängige Computer-Programme ist so eine Identifizierung potentiell antigener Sequenzen möglich.

### B.2 Untersuchungen zur Antigenität in vivo

Das Peptid mit der Aminosäure-Sequenz von BHV-1-Feldviren, die derjenigen entspricht, die in dem untersuchten BHV-1-Stamm als verändert identifiziert wurde, wird chemisch synthetisiert oder biochemisch gereinigt. Dieses Peptid wird im Tier, bevorzugt im Rind, auf Antigenität geprüft. Falls es sich bei dem Peptid um ein Hapten handelt, empfiehlt sich, vor der Applikation dieses Peptid an einen Carrier zu koppeln. Als Carrier seien genannt Bovines Serum Albumin, Ovalbumin oder KLH (Keyhole Limpet Hemocyanin). Die Methode der Bindung des synthetischen Peptides an einen Carrier erfolgt nach den an sich bekannten Methoden.

Die Tiere erhalten eine ein- oder mehrmalige Applikation des Peptides parenteral oder enteral, bevorzugt parenteral. Bevorzugt werden verschiedene Dosierungen des Peptides in verschiedene Tiergruppen appliziert. Als Dosierungen für das Peptid seien genannt 0,01 µg bis 100 mg, bevorzugt 0,1 µg bis 1 mg pro Applikation. In wöchentlichen bis monatlichen Abständen werden Serumproben der Tiere gewonnen und auf Antikörper gegen das applizierte Peptid untersucht. Als Methoden zur Untersuchung auf Antikörper seien genannt die gängigen immunologischen Testverfahren wie zum Beispiel Virusneutralisationstests, Enzyme-Linked-Immunoassays oder Immunoblots. Bevorzugt werden Enzyme-Linked-Imunosorbent-Assays (ELISA) eingesetzt, bei denen als Antigen das applizierte Peptid, das Protein, aus dem das Peptid stammt, oder intakte oder defekte BHV-1-Partikel eingesetzt werden.

Das applizierte Peptid kann als Antigen bezeichnet werden, wenn Tiere nach der Applikation des Peptides meßbar Antikörper bilden. Dies gibt einen Hinweis auf Eignung des untersuchten Stammes als Impfstamm gemäß 1 (oben).

Entscheidend ist, ob die entsprechende Aminosäure-Sequenz im Feldvirus, die in dem untersuchten BHV-1-Stamm als geändert identifiziert wurde, als Antigen im Rind erkannt wird.

Hierzu werden Rinder mit einem BHV-1, dessen Aminosäure-Sequenz nicht verändert ist, infiziert und nach der Infektion auf die Bildung von Antikörpern untersucht. Es werden in wöchentlichen oder monatlichen Intervallen Serumproben der Tiere genommen und immunologisch getestet. Bilden die Tiere nach der Infektion Antikörper gegen die Aminosäure-Sequenz des zur Infektion eingesetzten BHV-1,die derjenigen Sequenz entspricht, die in dem untersuchten BHV-1-Stamm als geändert identifiziert wurde, dann gilt die Antigenität der nicht geänderten Sequenz als belegt. Als immunologisches Nachweisverfahren zur Bestimmung der Antikörper gegen die nicht geänderte Aminosäure-Sequenz sei ein ELISA genannt, bei dem ein Peptid mit der gesamten oder mit Teilen der nicht-geänderten Aminosäure-Sequenz als Antigen benutzt wird.

Rinder, die mit dem untersuchten BHV-1-Stamm, der die geänderte Aminosäure-Sequenz enthält, ein oder mehrmals infiziert werden und/oder diesen als inaktiviertes Virus appliziert bekommen, dürfen keine Antikörper gegen die Aminosäure-Sequenz von Feldviren, die derjenigen entspricht die in dem untersuchten BHV-1 Stamm als geändert identifiziert wurde, bilden.

### B.3. Untersuchung von Rinderseren mit bekanntem BHV-1-Antikörper-Status

Rinderseren, deren Gehalt an BHV-1-spezifischen Antikörpern bekannt ist, werden auf den Gehalt an Antikörpern gegen die gesamte oder Teile der entsprechenden Aminosäure-Sequenz von BHV-1 Feldviren, die derjenigen Sequenz entspricht, die bei dem untersuchten BHV-1-Stamm als verändert identifiziert wurde, untersucht.

Ein untersuchter BHV-1-Stamm mit veränderter Aminosäure-Sequenz kann dann als erfindungsgemäßer Impfstamm gemäß 1) eingesetzt werden, wenn
1) in Rinderseren, die Antikörper gegen BHV-1 enthalten, ermittelt, z.B. im Virusneutralisationstest oder im ELISA auf Basis BHV-1 als Antigen, sich auch Antikörper gegen die nicht geänderte Aminosäure-Sequenz nachweisen lassen, und
2) Seren aus Rindern, die keine nachweisbaren Antikörper gegen BHV-1 enthalten, auch keine nachweisbaren Antikörper gegen die nicht geänderte Aminosäure-Sequenz enthalten.

### C. Prüfung auf Immunogenität gemäß 8 e

Die Identifizierung eines BHV-1-Stammes, der sich erfindungsgemäß als Virus-Stamm einer BHV-1-Marker-Vakzine eignet, schließt die Prüfung auf Immunogenität dieses Stammes ein.

Hierzu wird der untersuchte BHV-1-Stamm entweder als vermehrungsfähiges Virus (Lebendvakzine) oder aber als inaktiviertes Virus, in beiden Fällen eventuell formuliert mit einem Adjuvans, in Rinder appliziert. Die Applikation kann je nach Bedarf intramuskulär, subkutan, intratracheal, intranasal, intravaginal, intrapräputial, oder in den Konjunktivalsack erfolgen.

Der Impferfolg kann
- serologisch über die Induktion von BHV-1-spezifischen Antikörpern nach Immunisierung, ermittelt über Neutralisationstest oder ELISA, geprüft werden, oder
- über einen experimentellen Infektionsversuch mit virulentem BHV-1 ermittelt werden. Hierbei wird die klinische Manifestation nach einer experimentell gesetzten BHV-1-Infektion zwischen immunisierten und nicht immunisierten Rindern verglichen.

Geeignete Stämme verhindern oder reduzieren deutlich nach ein- oder zweimaliger Immunisierung bei Rindern die klinischen Manifestation der BHV-1-Infektion.

### D. Gentechnologische Herstellung gemäß 8f von BHV-1 Stämmen zur Verwendung gemäß 1

### D.1 Identifizierung von nicht-essentiellen Bereichen in essentiellen Proteinen von BHV-1

Die Identifizierung von für die Virusvermehrung nicht-essentiellen Bereichen in essentiellen Proteinen kann erfolgt durch
a) die Identifizierung von geänderten Aminosäure-Sequenzen in existenten BHV-1-Stämmen. Wie oben beschrieben, werden BHV-1-Stämme, die geänderte Aminosäure-Sequenzen tragen, über DNA- oder Aminosäure-Analysen identifiziert. Nach der Identifizierung werden die entsprechenden Genom-Sequenzen, falls nicht schon geschehen, molekular kloniert.
b) Modifikation von Genom-Sequenzen, die für Proteine kodieren, z.B. durch Austausch von Nukleotiden, Deletion von Nukleotiden oder Insertion von Nukleotiden, und anschließender Überprüfung der Bedeutung der Modifikation für die Vermehrungsfähigkeit des modifizierten Virus, wobei die untersuchte Genom-Sequenz und die daraus resultierende Aminosäure-Sequenz als nicht-essentiell für die Virusvermehrung anzusehen ist, wenn eine Veränderung der Aminosäuresequenz die Vermehrung des modifizierten Virus nicht verhindert.

### D.2 Nachweis der Antigenität, der identifizierten nicht-essentiellen Bereiche für Rinder nach Infektion mit BHV-1

### a) Theoretische Bewertung der Antigenität einer Aminosäure-Sequenz

Die theoretische Bewertung der in D.1 identifizierten Aminosäure-Sequenz erfolgt wie oben beschrieben.

### b) Untersuchung zur Antigenität in vivo

Nach chemischer Synthese des Peptids mit der entsprechenden Aminosäure-Sequenz von BHV-1, die derjenigen Sequenz entspricht, die als nicht-essentiell für die Virusvermehrung identifiziert wurde oder nach biochemischer Reinigung dieses Peptides aus BHV-1, wird dieses Peptid im Tier, bevorzugt im Rind, auf Antigenität geprüft. Die Prüfung im Tier erfolgt wie oben beschrieben.

Entscheidend ist, daß die in BHV-1 als nicht-essentiell identifizierte Aminosäure-Sequenz nach einer Infektion mit BHV-1-Feldvirus, als Antigen erkannt wird.

Hierzu werden Rinder mit BHV-1 infiziert und nach der Infektion auf die Bildung von Antikörpern untersucht. Hierzu werden in wöchentlichen oder monatlichen Intervallen Serumproben der Tiere gewonnen und immunologisch getestet. Bilden die Tiere nach der Infektion Antikörper gegen die oben identifizierte Aminosäuresequenz in BHV-1-Feldviren, dann gilt die Antigenität dieser Sequenz als belegt, Als immunologisches Nachweisverfahren zur Bestimmung der Antikörper gegen diese Aminosäure-Sequenz sei ein ELISA genannt, der als Antigen diese Aminosäure-Sequenz aus BHV1-Feldviren als Peptid trägt. Das Peptid kann z.B. chemisch synthetisiert werden oder biochemisch aus BHV-1-Feldviren gereinigt werden.

Rinder, die mit BHV-1 infiziert werden oder dieses als inaktiviertes Antigen erhalten, entwickeln auch nach mehrmaliger Applikation keine Antikörper gegen die in BHV-1 als nicht-essentiell identifizierte Aminosäure-Sequenz.

### D.3 Herstellung einer BHV-1-Mutante durch Veränderung der identifizierten Aminosäure-Sequenz durch Insertion, Substitution oder Deletion

Die identifizierte nicht-essentielle Region in BHV-1 kann durch Deletion, Insertion oder Substitution verändert werden. Hierzu werden die Genom-Fragmente, die die für diese nicht-essentielle Aminosäure-Sequenz kodierende DNA-Sequenz tragen, molekular kloniert. Anschließend werden aus diesen, bevorzugt in Vektoren klonierten DNA-Sequenzen, über die gängigen Methoden der Molekulargenetik die DNA-Sequenzen, die für die nicht essentiellen Aminosäure-Sequenzen kodieren, teilweise oder in ihrer ganzen Länge entfernt und/oder gegebenenfalls eine andere Nukleotid-Sequenz inseriert. Werden nur Teile eines für die Virus-Vermehrung essentiellen Proteins entfernt, so darf diese Deletion die Funktion des Proteins für die Virusvermehrung nicht blockieren. Der Einfluß der Veränderung der Aminosäure-Sequenz auf die Virusvermehrung wird in der Gewebekultur überprüft. BHV-1 Stämme, deren Aminosäure-Sequenz in nicht-essentiellen Teilen von essentiellen Proteinen verändert wurden und die gemäß (1) eingesetzt werden sollen, müssen sich in der Gewebekultur vermehren können.

Die inserierte Nukleotid-Sequenz darf die biologische Funktion des entsprechenden Proteins, in das sie inseriert wurde, da es sich um ein für die Virus-Vermehrung essentielles Protein handelt, ebenfalls nicht blockieren und kann für eine Aminosäure-Sequenz kodieren, über die das so veränderte Virus identifiziert werden kann. Die auf diese Weise durch Deletion und/oder Insertion veränderte DNA-Sequenz kann z.B. über Cotransfektion mit dem Genom von BHV-1 rekombiniert werden, um rekombinantes BHV-1 zu erhalten, das die nicht-essentielle Aminosäure-Sequenz in Teilen oder ganz deletiert trägt oder anstelle dieser eine neu, veränderte Aminosäure-Sequenz enthält.

### D.4. Erstellung der Korrelation des Vorkommens von Antikörpern gegen BHV-1 und gegen die identifizierte Aminosäure-Sequenz

### a) Untersuchung von Rinderseren mit bekanntem BHV-1-Antikörper-Status

Rinderseren, deren Gehalt an BHV-1-spezifischen Antikörpern bekannt ist, werden auf den Gehalt an Antikörpern gegen die oben identifizierte Aminosäure-Sequenz untersucht. Als Methode kann der oben genannte ELISA auf Basis eines Peptids mit der oben identifizierten Aminosäure-Sequenz als Antigen eingesetzt werden.

Eine als nicht-essentiell identifizierte Aminosäure-Sequenz kann dann als erfindungsgemäßer Marker einer BHV-1-Vakzine eingesetzt werden, wenn
1) in Rinderseren, die Antikörper gegen BHV-1 enthalten, ermittelt z.B. im Neutralisationstest oder im ELISA auf Basis BHV-1 als Antigen, sich auch Antikörper gegen die identifizierte Aminosäure-Sequenz nachweisen lassen, und
2) Seren aus Rindern, die keine nachweisbaren Antikörper gegen BHV-1 enthalten, auch keine nachweisbaren Antikörper gegen die identifizierte Aminosäure-Sequenz enthalten.

Als Test kann wieder der oben beschriebene ELISA auf Basis eines Peptides mit der identifizierten Aminosäure-Sequenz als Antigen eingesetzt werden.

### D.5. Prüfung auf Immunogenität

Die Prüfung erfolgt, wie oben beschrieben.

### I. Material und Methoden

### 1. Zell- und Virusmaterial

Als Zellsystem wurde eine permanente Rindernierenzelle (MDBK-Zelle) verwendet.
Folgende BHV-1 Stämme wurden in den Untersuchungen eingesetzt:
- BHV-1.1 Schleswig-Holstein (SH)
- BHV-1.2a Schönböken (SB)
- BHV-1.2b Australia 12 (Aus12)
- BHV-1.3 N569

Die Stämme SH, SB und Aus12 dienten als Beispiele für BHV-1 Feldviren.

### 2. Zell- und Virusvermehrung

Zur Zellvermehrung wurden 100 ml MDBK-Zellen mit 50x10³ Zellen/ml pro Rouxschale (175 cm²) inokuliert und in E-MEM + 0,85 g Bicarbonat/ltr. (Mayr et al. (1974). Virologische Arbeitsmethoden Bd. II. Gustav Fischer Verlag, Stuttgart) + 10 % FKS (fetales Kälberserum) vermehrt.

Zur Virusvermehrung wurde das Anzuchtmedium 3-5 Tage nach Zellaussaat vom konfluenten Zellrasen (ca. 500x10⁶ Zellen/Rouxschale) entfernt und durch E-MEM + 2,0 g Bicarbonat/ltr. (Mayr et al. (1974). Virologische Arbeitsmethoden Bd.II. Gustav Fischer Verlag, Stuttgart) ersetzt. Die Virusinfektion erfolgte mit einer Multiplizität der Infektion (MOI) von 0,01 bis 0,5. 3-4 Tage nach Virusinokulation, bei 100 % zpE (zytopathogenem Effekt), wurde das Virus geerntet.

### 3. Bestimmung des Virustiters

Zur Titerbestimmung von Virussuspensionen wurden 96-Lochplatten (Nunc) mit einem konfluenten MDBK-Zellrasen benutzt. Eine Verdünnungsreihe in Schritten zu logl0 10⁻¹ bis 10⁻⁹ des Virus, hergestellt in E-MEM + 2,0 g Bicarbonat, wurde mit 200µl/Vertiefung auf den Zellrasen ausgebracht. Die Titration wurde in 8-facher Bestimmung durchgeführt. Die Berechnung des erreichten Virustiters erfolgte nach Spearman und Kärber (Mayr et al. (1974). Virologische Arbeitsmethoden Bd.I. Gustav Fischer Verlag, Stuttgart.).

### 4. Darstellung der BHV-1 Strukturproteine

Für die Darstellung viraler Proteine wurden konfluente MDBK-Zellen infiziert und von 6 bis 20 h p.i. mit ³⁵S-Methionin metabolisch markiert. Das virale gIV wurde aus infizierten Zellen mit Hilfe eines monospezifischen anti-gIV Serums präzipitiert, in einem 10% SDS-Polyacrylamidgel nach Lämmli aufgetrennt und nach Fluorgraphie in der Autoradiographie sichtbar gemacht. In diesem Versuchsansatz lag gIV schon vorwiegend glykosyliert vor. Es wurden nur "reife" Formen des gIV präzipitiert.

### 5. Reinigung viraler DNA

Rouxschalen mit einem konfluenten MDBK-Zellrasen wurden mit Virus beimpft und 3-4 Tage bei +37°C bebrütet. Bei 100 % zpE wurde die gesamte Virussuspension mit 5000 xg für 20 Minuten zentrifugiert, das entstandene Pellet in PBS (140 mM NaCl; 2,7 mM KCI; 6,5 mM Na₂HPO₄; 0,7 mM CaCl₂; 0,5 mM MgCl₂; 1,5 mM KH₂PO₄) resuspendiert und erneut abzentrifugiert (10000 xg; 20 Minuten). Die Überstände wurden vereinigt, danach 1 h bei 100000 xg zentrifugiert und das Viruspellet in 100 µl über Nacht resuspendiert. Danach wurde das Viruspellet ad 5 ml PBS mit 5mM MgCl₂ resuspendiert, homogenisiert, anschließend mit DNAseI (Endkonzentration 100 µg/ml) versetzt und 1 h bei +37°C inkubiert. Nach der DNAse-Behandlung wurden die Viruspartikel für 1 h bei 100000 xg durch ein 15%iges Saccharosekissen zentrifugiert. Anschließend wurde das Pellet in 1,8 ml 20 mM Tris-Puffer pH 8,0 resuspendiert und nach Zugabe von 200 µl 20%igem Sarcosyl 1h bei +56°C inkubiert. Die virale DNA wurde 48 h bei 300000 xg in einem Festwinkelrotor bei 20°C in einem CsCl-Gleichgewichtsgradienten (5,7 mM CsCl₂; 10 mM Tris pH7; 100 mM EDTA) zentrifugiert, der Gradient fraktioniert und Aliquots in einem 0,6 % Agarosegel auf DNA-Gehalt untersucht. DNA-haltige Fraktionen wurden vereinigt, nochmals in einem CsCl-Gleichgewichtsgradienten zentrifugiert und gegen 20 mM Tris-Puffer pH 8,0 dialysiert. Die DNA-Konzentration wurde im Photometer bei 260 nm bestimmt.

### 6. Klonierung und Sequenzierung von gIV

### 6.1 Klonierung

Die gereinigte virale DNA vom BHV-1 Stamm SB wurde mit HindIII restringiert (Boehringer-Mannheim, nach Angaben des Herstellers). Anschließend wurden die entstandenen DNA-Fragmente in einem 0,6 % Agarosegel in TA-Puffer (33 mM Tris, 66 mM Kaliumacetat, 10 mM MG-Acetat, pH 7,9 mit Eisessig, 0,1 mg/ml BSA, 0,5 mM DDT) elektrophoretisch getrennt und das HindIII-L-Fragment mit 6,6 kbp elektroeluiert (Sambrook et al. (1989). Molecular Cloning. Cold Spring Harbor Laboratory Press.). Unter Einsatz der T4-Ligase wurde das HindIII-L-Fragment in die HindIII-Schnittstelle des Vektors pUC12 inseriert. Hierzu wurde die DNA des Vektors mit HindIII behandelt, die Rezirkularisierung durch Abspaltung der 5'-phosphatgruppe mittels alkalischer Phosphatase verhindert, und die linearisierte DNA des Vektors pUC12 mit dem isolierten HindIII-Fragment und T4-Ligase inkubiert.

Beim Stamm Aus12 wurde das mit dem HindIII-L-Fragment des Stammes SB kreuzhybridisierende 8,0 kbp HindIII-DNA-Fragment in der oben beschriebenen Weise isoliert und ebenfalls mit der linearisierten und mit HindIII behandelten DNA des Vektors pUC12 und der T4-Ligase inkubiert.

Anschließend wurden die Plasmide in E.coli C600 transfiziert (Sambrook et al. (1989). Molecular Cloning. Cold Spring Harbor Laboratory Press.) und Bakterienklone mit rekombinanten Plasmiden, anhand ihrer Ampicillinresistenz und fehlender lacZ-Aktivität, selektiert (Sambrook et al. (1989). Molecular Cloning. Cold Spring Harbor Laboratory Press.).

Zur Sequenzierung wurden die Bakterien mit den rekombinanten Plasmiden vermehrt und die Plasmid-DNA gereinigt (Sambrook et al. (1989) Molecular Cloning. Cold Spring Harbor Laboratory Press). Anschließend wurde die DNA mit PstI gespalten und die Spaltprodukte wiederum in einem 0,6 % Agarosegel elektrophoretisch getrennt und elektroeluiert (Sambrook et al. (1989). Molecular Cloning. Cold Spring Harbor Laboratory Press). Die isolierten DNA-Fragmente wurden in den Sequenzier-Vektor pEMBL 19 inseriert und wiederum in E.coli C600 vermehrt (Sambrook et al. (1989). Molecular Cloning. Cold Spring Harbor Laboratory Press.).

Zur Identifizierung des gIV-Gens beim Stamm N569 wurde dessen genomische DNA mit PstI gespalten und die Fragmente in einem 0,6 % Agarosegel in TA-Puffer elektrophoretisch getrennt. Anschließend wurden die DNA-Fragmente auf eine Nitrocellulose-membran im Southern Blot transferriert (Sambrook et al. (1989). Molecular Cloning. Cold Spring Harbor Laboratory Press) und mit dem radioaktivmarkierten HindIII-L-Fragment des Stammes SB hybridisiert (Sambrook et al. (1989). Molecular Cloning. Cold Spring Harbor Laboratory Press.). Mittels der Hybridisierungsreaktion wurde ein 10,1 kbp großes Fragment als Träger des gIV-Gens bei N569 identifiziert. Nach Identifizierung des gIV-Gens wurde das entsprechende 10,1 kbp-Fragment mittels Gelelektrophorese und Elektroelution isoliert und unter Einsatz der T4-Ligase analog der Vorgehensweise bei den Stämmen SB und Aus12 in die PstI-Schnittstelle des Vektors pUC12 inseriert. Hierzu wurde die DNA des Vektors mit PstI behandelt, die Rezirkularisierung durch Abspaltung der 5'-Phosphatgruppe mittels alkalischer Phosphatase verhindert, und die linearisierte DNA des Vektors pUC12 mit dem isolierten 10,1 kbp-Fragment und T4-Ligase inkubiert.

### 6.2 Sequenzierung

Die Sequenzierung der PstI-Fragmente erfolgte nach einer Methode, die auch als "chromosomen-walking" bezeichnet wird. Sie ist gut geeignet, um von einem bestehenden Klon, ohne weitere Subklonierung, große Inserts schnell zu sequenzieren. Dabei wurden synthetische Oligonukleotide als Primer eingesetzt, die an unterschiedlichen Stellen des Inserts hybridisierten und dort die Sequenzreaktion nach Sanger gestartet. Dazu wurden die Fragmente von der Flanke der PstI-Spaltstelle her sequenziert. Gegen das Ende dieser Sequenz wurde dann ein hybridisierendes Oligonukleotid hergestellt und an Stelle des Universal-Primers verwendet. Mit diesem neuen Primer wurde dann weiter in die Fragmente sequenziert und danach der obengenannte Vorgang wiederholt. Da die erhaltenen Sequenzen in einigen Bereichen nicht überlappten, wurden die Fragmente zusätzlich mit dem ExoIII/S1-System gezielt verkürzt, ligiert und in E.coli C600 kloniert. Diese Subklone wurden sequenziert und die Sequenzen ergaben die benötigten Überlappungen zu den bereits vorhandenen Sequenzen.

### 7. Bestimmung serumneutralisierender Antikörper gegen BHV-1

Der Serumneutralisationstest zur Bestimmung neutralisierender BHV-1 Antikörper wurde gemäß der Vorschrift aus den Virologischen Arbeitsmethoden Bd.II (Mayr et al. (1974). Gustav Fischer Verlag, Stuttgart.) durchgeführt.

### 8. Kopplung des Peptids an BSA als Trägerprotein

### 8.1 Pep2

Das synthetisch hergestellte Pep2 besitzt die Aminosäure-Sequenz des Peptidsegments AS317 bis AS333 des gIV vom BHV-1 Stamm Schönböken:

gly-gly-ala-glu-gly-pro-lys-pro-gly-pro-ser-pro-
asp-ala.

### 8.2 Pep3

Das ebenfalls chemisch synthetisierte Pep3 besitzt neben den 15 Aminosäuren des Pep2 weitere 15 Aminosäuren des gIV von Schönböken. Es umfaßt die Sequenz des Peptidsegments von AS307 bis AS338 im gIV des BHV-1 Stammes SB:

asp-gly-glu-ser-gln-thr-pro-glu-ala-asn-gly-gly-
ala-glu-gly-glu-pro-lys-pro-gly-pro-ser-pro-asp-
ala-asp-arg-pro-glu-gly.

### 8.3 Kopplung von snythetischem Peptid an BSA

5 mg synthetisch hergestelltes Peptid (Pep2 oder Pep3) wurden in 1 ml PBS pH=7,4 gelöst und auf pH 7,0 eingestellt. Im Falle des Pep2 wurden 11 mg BSA, bei Pep3 6,2 mg BSA in 1 ml PBS gelöst und unter starkem Rühren zur Peptidlösung gegeben. Anschließend wurden 2 ml einer 0,2 % Glutaraldehydlösung in PBS unter ständigem Rühren tropfenweise zu dem Peptid-BSA-Gemisch zugegeben. Der gesamte Ansatz wurde über Nacht bei Raumtemperatur inkubiert und am nächsten Tag 0,8 ml einer 1M Glycinlösung in PBS hinzupipetiert, 1 Stunde gerührt und danach das Konjugat für 5 Stunden gegen PBS (pH=7,4) dialysiert, portioniert und bei -20°C eingefroren.

### 9. ELISA zur BHV-1 Antikörper-Bestimmung (VV-ELISA) in Rinderseren

Im Vollvirus-ELISA wurde als Antigen gradientengereinigte Viruspartikel des BHV-1 Stammes SH eingesetzt. Die Immulon 96-Lochplatte (F-Form, Dynatech) wurde mit 50 ng Antigen in 50 µl Coating-Puffer (1,06 g Na₂CO₃, 2,93 g NaHCO₃ ad 1,01 Aqua dest., pH=9,6) pro Vertiefung beschichtet. Danach wurden die Platten für 16 Stunden bei +37°C im CO₂-Brutschrank inkubiert. Nach 3-maligem Waschen mit Waschpuffer (PBS + 0,05 % Tween 20 + 1M NaCl) wurden die Platten, außer dem Leerwert, mit Blockpuffer (200 µl PBS/Tween + 1M NaCl + 2 % Ovalbumin) beschickt. Sowohl die Platten, als auch die Testseren 1:100 verdünnt in Blockpuffer, wurden für 1 Stunde bei 37°C inkubiert. Nach 3-maligem Waschen mit Waschpuffer wurden 100µl Serum pro Napf (1:100 bis 1:51200 in 2er Reihe verdünnt) in Doppelbestimmung in die Platte pipetiert und die Platten anschließend für 2 Stunden bei +37°C in einer feuchten Kammer inkubiert. Nach gründlichem Waschen der Platten wurden diese mit 50µl POD-gekoppeltem Kaninchen-Anti-Rind-Konjugat pro well (1:2000 verdünnt in PBS + 0,05 % Tween 20) beschickt. Die Platten wurden wieder für 2 Stunden bei +37°C inkubiert, gewaschen, mit 100µl Substratlösung (11 mg ABTS gelöst in 2,1 g Zitronensäure auf 100 ml Aqua dest., pH = 4,2) beschickt und 60' im Dunkeln inkubiert, Das Messen der optischen Dichte erfolgte bei 405 nm. Die Serumverdünnung die im ELISA zu einer Extinktion von 0,1 führte, wurde als Antikörpertiter definiert. Seren mit einem Titer > 1:1000 wurden als BHV-1 positiv bewertet.

Bei jedem ELISA wurden folgende Kontrollen angelegt:

| Kontrolle | Antigen | 1.AK | 2.AK | Substrat |
|---|---|---|---|---|
| Leerwert | nein | nein | nein | ja |
| Antigenkontrolle | ja | nein | ja | ja |
| Antikörperkontrolle | nein | ja | ja | ja |
| Konjugatkontrolle | nein | nein | ja | ja |

### 10. ELISA mit synthetischem Peptid (Pep2) als Antigen für Rinderseren zur Quantifizierung von Antikörpern gegen gD von BHV-1 (Pep-ELISA)

Im Pep-ELISA wurde die Immulon 96-Lochplatte in F-Form, der Fa. Dynatech, eingesetzt. Als Antigen wurde Pep2 gekoppelt an BSA (siehe Punkt 6) 100ng/well, in 50µl Coatingpuffer, eingesetzt.

Die beschichteten Platten wurden 16 Stunden bei +37°C in einer feuchten Kammer inkubiert. Nach 3-maligem Waschen mit Waschpuffer (PBS + 0,05 % Tween 20) wurden alle Vertiefungen mit 200µl Blockpuffer (PBS + 0,05 % Tween 20 + 1 % Magermilchpulver beschickt. beziehungsweise die Testseren 1:100 in Blockpuffer + 1,5M NaCl verdünnt. Sowohl die geblockten Platten als auch die verdünnten Seren wurden 1 Stunde bei +37°C inkubiert. Danach wurden je 200µl der zu testenden Seren, 1:100 bis 1:3200 verdünnt in Blockpuffer + 1,5M NaCl, in die Platten einpipetiert und die Platten für 2 Stunden bei +37°C inkubiert. Anschließend wurden die Platten 3 x mit Waschpuffer gewaschen und 50µl Kaninchen-Anti-Rind-POD-Konjugat (1:2000 verdünnt in PBS + 0,05 % Tween 20) in jede Vertiefung zugegeben, 2 Stunden bei +37°C inkubiert, die Platten wieder 3 x mit Waschpuffer gewaschen und 100µl Substratlösung (11mg ABTS gelöst in 98ml Substratpuffer (2,1g Zitronensäure ad 98ml mit Aqua dest., pH=4,2)) pro Vertiefung einpipetiert. Die Farbreaktion wurde nach einstündiger Inkubation bei +37°C und Dunkelheit mit 100µl 1%iger SDS-Lösung gestoppt. Die Messung erfolgte bei 405nm.

Bei jedem ELISA wurden folgende Kontrollen angelegt:

| Kontrolle | Antigen | 1.AK | 2.AK | Substrat |
|---|---|---|---|---|
| Leerwert | nein | nein | nein | ja |
| Antigenkontrolle | ja | nein | ja | ja |
| Antikörperkontrolle | nein | ja | ja | ja |
| Konjugatkontrolle | nein | nein | ja | ja |

### Bewertung:

Seren,bei denen die Extinktion der 1:100 Verdünnung abzüglich der Extinktion der Antikörperkontrolle ≥ 0,100 O.D betrug, wurden als BHV-1 positiv bewertet.

Seren, bei denen die Extinktion der 1:100 Verdünnung abzüglich der Antikörperkontrolle < 0,100 O.D betrug, wurden als BHV-1 negativ bewertet.

### 11. Dotblot

Vor dem Proteinauftrag wurde die Trägermembran (Immobilien P, Millipore) kurz mit Methanol benetzt und anschließend mit reichlich Aqua dest. gewaschen und bis zum Gebrauch in PBS (pH 7,4) aufbewahrt. 0,5µg und 1,0µg synthetisches Peptid (Pep3 gekoppelt an BSA), beziehungsweise 1,0µg BSA jeweils in 100µl PBS wurden auf den Membranfilter aufgetragen.

Die freien Bindungsstellen der Immobilienmembran wurden anschließend für 2 Stunden mit Blockpuffer abgedeckt (PBS + 0,5% Magermilchpulver + 0,05% Tween). Danach wurde die Membran für 2 Stunden mit dem 1. Antikörper (1:50 verdünnt in PBS + 0,05% Tween) inkubiert, 3 x gewaschen (PBS + 0,05% Tween), und für weitere 2 Stunden mit dem 2. Antikörper (Anti-Rind-POD-Konjugat; Sigma) (1:2000 verdünnt in PBS + 0,05% Tween) inkubiert und wieder 3 x in PBS + 0,05% Tween gewaschen. Alle Inkubationsschritte wurden bei Raumtemperatur durchgeführt. Nach dem letzten Waschgang wurde frisch angesetztes Substratgemisch:
- 20 mM NaCH₃COO-Lösung, pH 5,0-5,5
- 10 mg 3-Amino-9-Ethylcarbazol gelöst in 3ml DMSO
- 0,04 ml einer 30%igen H₂O₂-Lösung
zugegeben und die Färbung nach 15 Minuten ausgewertet. Die Bindung von Pep3-spezifischen Antikörpern an Pep3 wurde somit durch eine Farbreaktion auf der Membran signalisiert.

### 12. Rinderseren

Für die serologischen Untersuchungen im Pep-ELISA, VV-ELISA, SNT und Dotblot standen Rinderseren von Tieren, die mit folgenden BHV-1 Stämmen infiziert wurden, zur Verfügung:
- Australia12
- N569
- Schönböken
- Schleswig-Holstein

Außerdem wurden in Pep-ELISA, VV-ELISA und SNT Feldseren aus Norddeutschland mit bekanntem Gehalt an BHV-1 spezifischen Antikörpern, geprüft im ELISA, eingesetzt.

### II. Ergebnisse

### 1. Größenheterogenität im gIV

In den folgenden Untersuchungen wurde das gIV der BHV-1 Stämme SB, Aus12 und N569, wie unter Punkt 4 beschrieben dargestellt, auf sein Größe in der Gelelektrophorese untersucht. Die Molekulargewichte der Proteine werden abgeleitet von ihrem Laufverhalten im Gel.

In den gelelektrophoretisch untersuchten Proben zeigten sich deutliche Unterschiede in der Größe des gIV bei den Stämmen Aus12, SB und N569. Die Größe des gIV lag bei Aus12 bei 84KD, für SB bei 72KD und N569 bei 68KD.

Eine Größenheterogenität kann theoretisch durch unterschiedlich große "open reading frames" (ORF) im gIV-Gen der verschiedenen BHV-1 Stämme verursacht werden. Andere Möglichkeiten sind die unterschiedlichen co- und posttranslationalen Modifikationen des gIV.

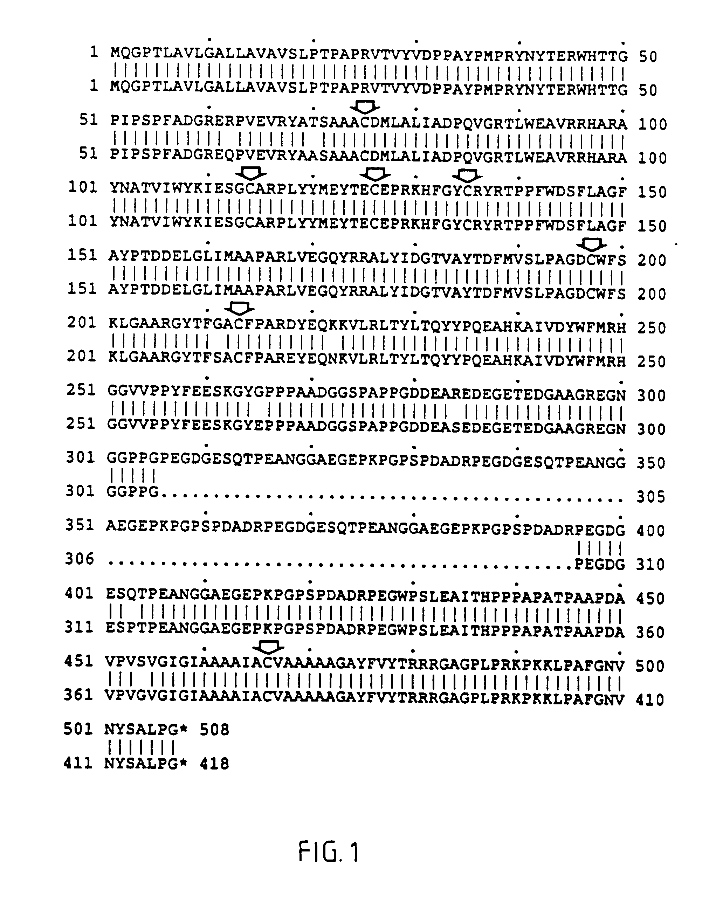

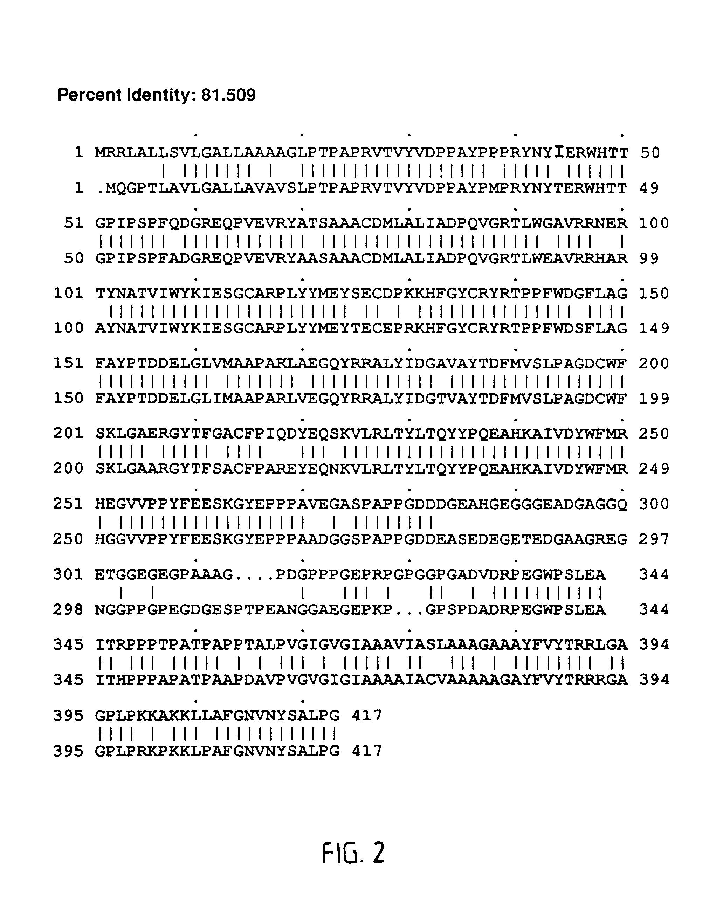

Zur Charakterisierung des ORF des gIV bei diversen BHV-1 Stämmen wurde die Gesamtsequenz des gIV-Gens für die Stämme Aus12, N569 und SB bestimmt, miteinander verglichen und die Aminosäuresequenz des Proteins daraus abgeleitet. Abb. 1 zeigt vergleichend die Aminosäure-Sequenz des gIV für die Virusstämme Aus12 und SB, Abb. 2 für die Stämme N569 und SB.

Bei Aus12 umfaßt der ORF des gIV 1524 bp die für 507 Aminosäuren codieren, bei SB 1254 bp die für 417 Aminosäuren codieren. Für die Größenheterogenität des ORF in den gIV-Genen ist eine repetetive Sequenz verantwortlich. Diese 90 bp große repeat-Sequenz liegt als direkte Wiederholung beim Stamm Aus12 in 4facher Kopienzahl, beim Stamm SB in 1facher Kopienzahl vor. Im Stamm SB ist diese repeat-Sequenz von bp 1022 bis bp 1111 des ORF lokalisiert und codiert für die Aminosäuren 306-335 mit folgender Sequenz:

P-E-G-D-G-E-S-Q-T-P-E-A-N-G-G-A-E-G-E-P-K-P-G-P-S-
P-D-A-D-R.

Bei Aus12 reicht diese repeat-Sequenz von bp 1318 bis bp 1408 des ORF und codiert für die Aminosäuren 306-425, einer 4fach Kopie der Sequenz vom Stamm SB.

Der Stamm N569 besitzt im Bereich der repeat-Sequenz, die bei ihm genau wie bei SB nur in einfacher Kopie vorliegt, eine andere Aminosäuresequenz als die Stämme SB und Aus12.

In diesem Bereich bestehen zwischen den beiden untersuchten Stämmen SB und N569 lediglich 19% Homologie verglichen mit 81,5% Homologie bezogen auf das gesamte gIV-Protein dieser beiden BHV-1 Stämme. Die Sequenz bei N569 im Bereich des Repeat lautet:

E-G-P-A-A-A-G-P-D-G-P-P-P-G-E-P-R-P-G-P-G-G-P-G-A-
D-V-D-R.

Da sowohl bei SB als auch bei N569 das gIV 417 Aminosäuren umfaßt, kann die in elektrophoretisch untersuchten Proben beobachtete Größenheterogenität im gIV dieser beiden Stämme nicht auf unterschiedlich großen ORFs beruhen. Vielmehr ist beim Stamm N569 in Position 44 die Aminosäure Threonin durch die Aminosäure Isoleucin substituiert. Der damit verbundene Verlust einer Glykosilierungsstelle ist vermutlich die Ursache für die in der Gelelektrophorese beobachtete Größenheterogenität im gIV bei SB und N569.

### 2. Computeranalyse

Mittels der "Chou-Fosman Prediction" für das gIV-Protein des Stammes SB wurde die repeat-Region als potentielles Antigen (hydrophile Region) identifiziert.

### 3. Antigenität im Rind

Rinderseren, genommen vor und nach Infektion mit den BHV-1 Stämmen Aus12, SB, SH und N569, beziehungsweise Feldseren von Rindern mit bekanntem BHV-1 Antikörpergehalt, wurden im Pep-ELISA auf Pep2-spezifische Antikörper, im VV-ELISA und SNT auf BHV-1-spezifische Antikörper und im Dotblot auf Pep3-spezifische Antikörper untersucht.

### 3.1 Australia-12-Seren

Tab. 1 zeigt vergleichend die Ergebnisse von 18 Rinderseren aus 7 Tieren vor und nach Infektion mit dem BHV-1 Stamm Aus12. Zur Charakterisierung wurden die Seren im Pep-ELISA, VV-ELISA, SNT und Dotblot auf Pep- und BHV-1-spezifische Antikörper untersucht. In allen Rinderseren waren vor Belastungsinfektion im SNT, VV-ElISA, Pep-ELISA und Dotblot keine gegen BHV-1 Viruspartikel, Pep2 und Pep3 gerichteten Antikörper nachweisbar.

Mit dem SNT konnte in allen Seren nach Infektion, außer im Serum der OM 25123 nach Erst infektion und im Serum der OM 25126 nach Boosterinfektion, virusneutralisierende BHV-1 Antikörper nachgewiesen werden. Bei der OM 25123 waren erst nach Boosterinfektion neutralisierende BHV-1 Antikörper zu detektieren.

Mit dem VV-ELISA konnte in denselben Seren nach Infektion, in denen virusneutralisierende Antikörper nachzuweisen waren, ebenfalls BHV-1-spezifische Antikörper detektiert werden, mit Ausnahme des Serums der OM 25124 nach Erstinfektion.

Bei den Seren, die serumneutralisierende Antikörper zeigten, waren ebenfalls gegen das Pep2 gerichtete Antikörper im ELISA nachzuweisen. Eine Ausnahme war das Serum der OM 87 nach Infektion, das zwar Virusneutralisierende BHV-1 Antikörper im SNT, aber keine Pep2-Antikörper im ELISA besaß.

Im Serum des Rindes OM 25123 waren nach der 1. Infektion und im Serum des Rindes OM 25126 waren nach Reinfektion sowohl im Serumneutralisationstest als auch im Pep-ELISA keine BHV-1 und Pep2-spezifischen Antikörper zu detektieren.

In Seren, die nach der 1. Infektion mit BHV-1 genommen wurden und die virusneutralisierende Antikörper besaßen, waren mit Hilfe des Dotblots gegen das Pep3 gerichtete Antikörper nachzuweisen. Zusätzlich konnte mit dem Dotblot im Serum der OM 25123, das nach Infektion genommen wurde (vom 21.09.90), und das keine serumneutralisierenden und ELISA-Antikörper enthielt, gegen das Pep3 gerichtete Antikörper nachgewiesen werden.

### 3.2 N569 Seren

Tab. 2 zeigt, analog zu Tab. 1, vergleichend die Ergebnisse von Rinderseren vor und nach Infektion mit dem BHV-1 Stamm N569. Zur Charakterisierung wurden die Seren im Pep-ELISA, VV-ELISA und SNT auf Pep2- beziehungsweise BHV-1 Viruspartikel spezifische Antikörper untersucht.

Vor der Infektion mit dem BHV-1 Stamm N569 waren in keinem der Rinderseren sowohl im SNT, VV-ELISA als auch im Pep-ELISA gegen BHV-1 oder Pep2 gerichtete Antikörper zu detektieren. Im Anschluß an die Infektion waren im SNT und VV-ELISA in beiden Seren BHV-1-spezifische Antkörper nachweisbar. Gegen Pep2 gerichtete Antikörper waren jedoch auch nach Belastungsinfektion im Pep-ELISA nicht zu finden.

Somit bilden die mit N569 infizierten Rinder zwar im VV-ELISA und SNT reagierende BHV-1-spezifische Antikörper aus, erkannten im Pep-ELISA post infectionem jedoch nicht, das im gIV der Stämme SB und Aus12 nachgewiesene, beim N569 jedoch nicht identifizierte Pep2 als Antigen.

### 3.3 Schleswig-Holstein- und Schönböken-Seren

Tab. 3 zeigt vergleichend die Ergebnisse von Rinderseren vor und nach Infektion mit den BHV-1 Stammen SB und SH. Auch diese Seren wurden zur Charakterisierung im Pep-ELISA, VV-ELISA, SNT und Dotblot auf Pep- und BHV-1-spezifische Antikörper untersucht. Vor Infektion mit den BHV-1 Stämmen SH und SB war in keinem getesteten Serum, nach Infektion in allen Rinderseren gegen das BHV-1, Pep2 und Pep3 gerichtete Antikörper im SNT, VV-ELISA und Pep-ELISA zu detektieren.

### 3.4 Feldseren

Tab. 4 zeigt die Ergebnisse der Untersuchungen von Rinderseren aus kontrollierten Beständen, deren BHV-1 Antikörperstatus im ELISA geprüft worden war. Die Seren wurden im Pep2-ELISA und VV-ELISA auf BHV-1 und Pep2-spezifische Antikörper untersucht.

In 29 von den 31 getesteten Positivseren konnte mit dem VV-ELISA BHV-1-spezifische Antikörper detektiert werden. Im Pep-ELISA konnte in allen 31 Positivseren gegen das Pep2 gerichtete Antikörper gefunden werden.

In den Negativseren konnten im VV-ELISA bei den 6 getesteten, im Pep-ELISA bei 5 der 6 geprüften Seren keine BHV-1- und Pep2-spezifischen Antikörper nachgewiesen werden.

### 4. Prüfung des Stammes N569 auf Immunogenität

Tab. 5 zeigt die Ergebnisse der Untersuchungen von Rinderseren im SNT nach Immunisierung mit dem Stamm N569. Die 4 Rinder wurden in unterschiedlicher Weise mit dem Stamm N569 immunisiert.

Spätestens 13 Tage nach Immunisierung waren serum-neutralisierende Antikörper in jedem Serum der geimpften Tiere nachzuweisen. Den höchsten Antikörpertiter erreichte das intravenös (i.v.) geimpfte Rind mit einem Titer 1:152. Nach intramuskulärer (i.m.) und intranasaler (i.n.) Applikation des Stammes N569 wurden Antikörperspiegel von 1:45 und 1:27 erreicht, nach intravaginaler (i.vag.) Applikation lag der erreichte Antikörperspiegel bei 1:9.

### 5. Fazit

Durch den kombinierten Vergleich, der gegen die geänderte Aminosäuresequenz des Stammes N569 gerichteten Antikörper von gegen BHV-1-Viruspartikel gerichteten Antikörpern, konnte zwischen BHV-1-Antikörper freien Rinder, mit dem Stamm N569 immunisierten Tieren und mit Feldstämmen infizierten Rindern differenziert werden.

Nach i.v., i.m., i.n. und i.vag. Applikation erwies sich der BHV-1 Stamm N569 als immunogen für das Rind.

### Beschreibung der Abbildungen:

Abb, 1 zeigt vergleichend die Aminosäure-Sequenzen des gIV der BHV-1 Stämme Aus12 und Schönböken. Die Aminosäure-Sequenz und Homologien wurden durch das PC-Programm PC-Gen identifiziert. Die obere Sequenz gehört zum Stamm Aus 12, die untere Sequenz zum Stamm Schönböken. Die Sequenzen beginnen am 5'-Ende (N-Terminus) des gIV und enden am 3'-Ende (C-Terminus). Striche zwischen den beiden Strängen zeigen Homologien an. Die eingezeichneten Pfeile markieren die Aminosäure Cystein.

Abb. 2 zeigt vergleichend die Aminosäure-Sequenzen des gIV der BHV-1 Stämme N569 und Schönböken. Die Aminosäure-Sequenz und Homologien wurden durch das PC-Programm PC-Gen identifiziert. Die obere Sequenz gehört zum Stamm N569, die untere Sequenz zum Stamm Schönböken. Die Sequenzen beginnen am 5'-Ende (N-Terminus) des gIV und enden am 3'-Ende (C-Terminus). Striche zwischen den beiden Strängen zeigen Homologien an. Unterstrichen ist die Sequenz des synthetisierten Pep3. Das Kästenchen in Position AS41-43 markiert die fehlende Glykosilierungsstelle des BHV-1 Stammes N569.

Tab. 1 zeigt vergleichend die Ergebnisse von Rinderseren vor und nach Infektion mit dem BHV-1 Stamm Aus12. Zur Charakterisierung wurden die Seren im Pep-ELISA, VV-ELISA, SNT und Dotblot auf Pep- beziehungsweise BHV-1-spezifische Antikörper untersucht.

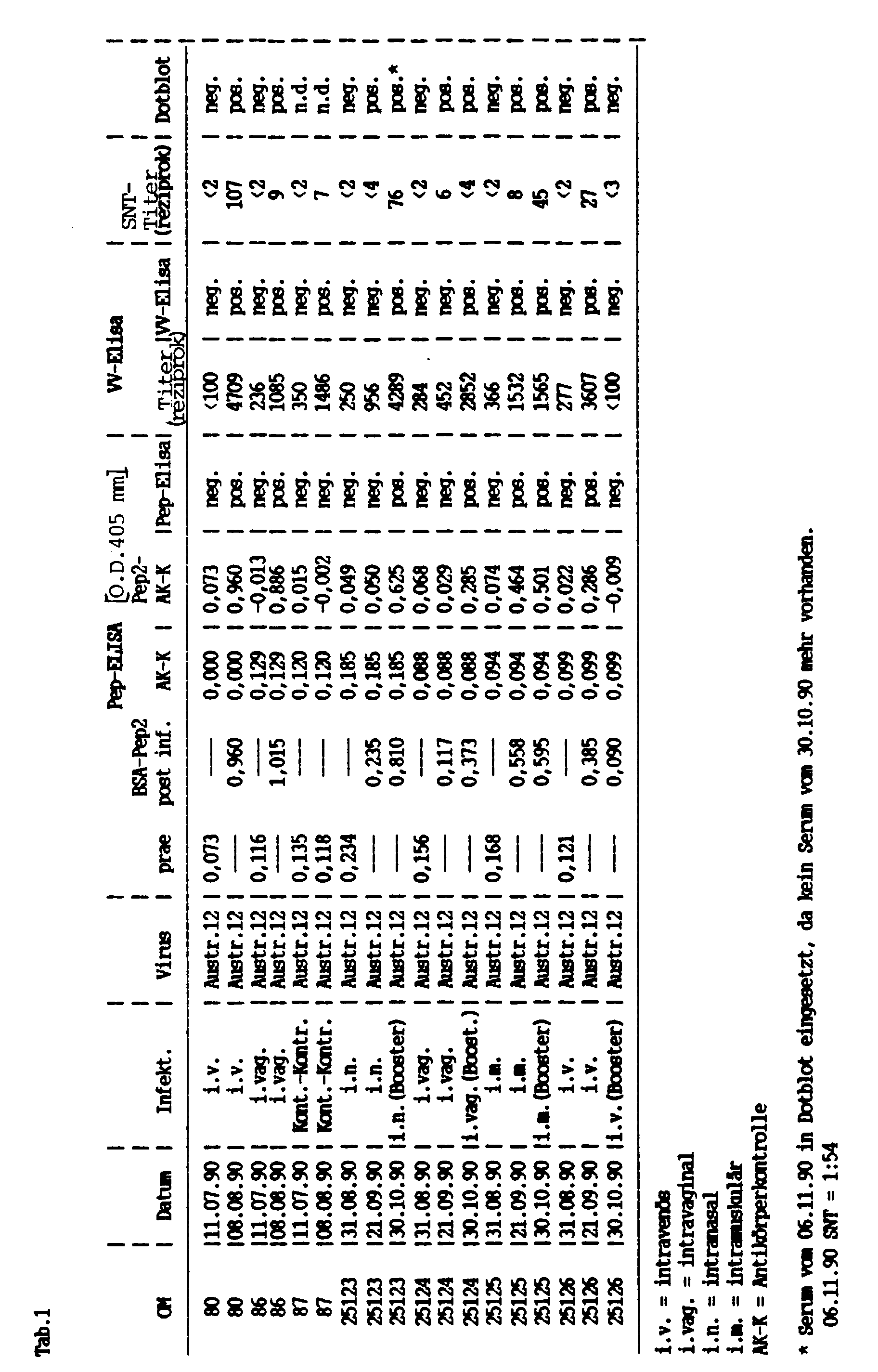

Tab. 2 zeigt vergleichend die Ergebnisse von Rinderseren vor und nach Infektion mit dem BHV-1 Stamm N569. Die Seren wurden zur Charakterisierung im Pep-ELISA, VV-ELISA und SNT auf Pep- beziehungsweise BHV-1-spezifische Antikörper untersucht.

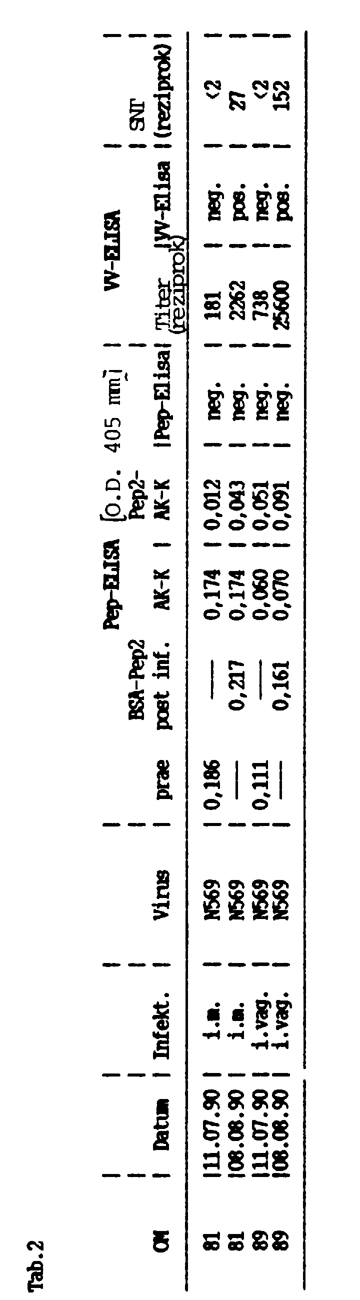

Tab. 3 zeigt vergleichend die Ergebnisse von Rinderseren vor und nach Infektion mit den BHV-1 Stämmen SB und SH. Die Seren wurden zur Charakterisierung im Pep-ELISA, VV-ELISA, SNT und Dotblot auf Pep- beziehungsweise BHV-1-spezifische Antikörper untersucht.

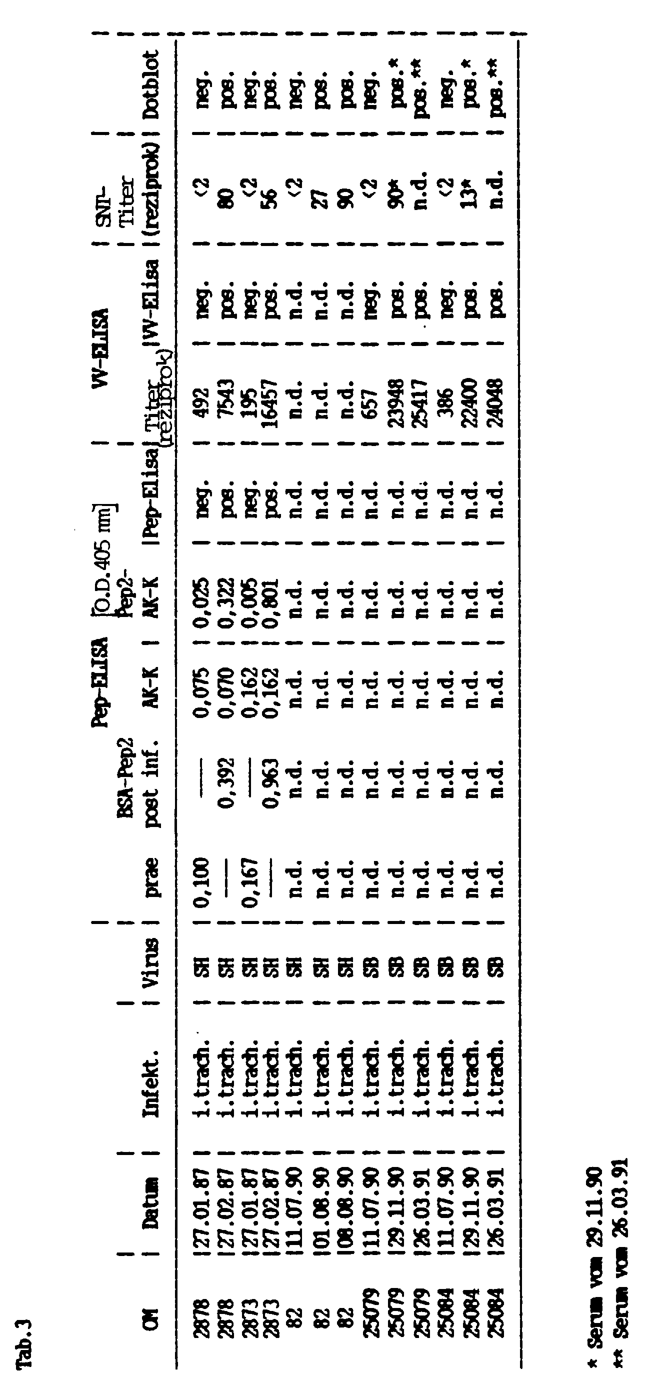

Tab. 4 zeigt die Ergebnisse von Pep2-ELISA, VV-ELISA und SNT mit Feldseren mit bekannten BHV-1-Status. Für die Untersuchungen standen 31 Positivseren und 6 Negativseren deren BHV-1-Antikörperstatus vom Tiergesundheitsdienst Oldenburg mittels ELISA bestimmt worden waren zur Verfügung. Alle Seren wurden im Pep-ELISA und VV-ELISA charakterisiert. Im SNT wurden 3 Positivseren getestet.

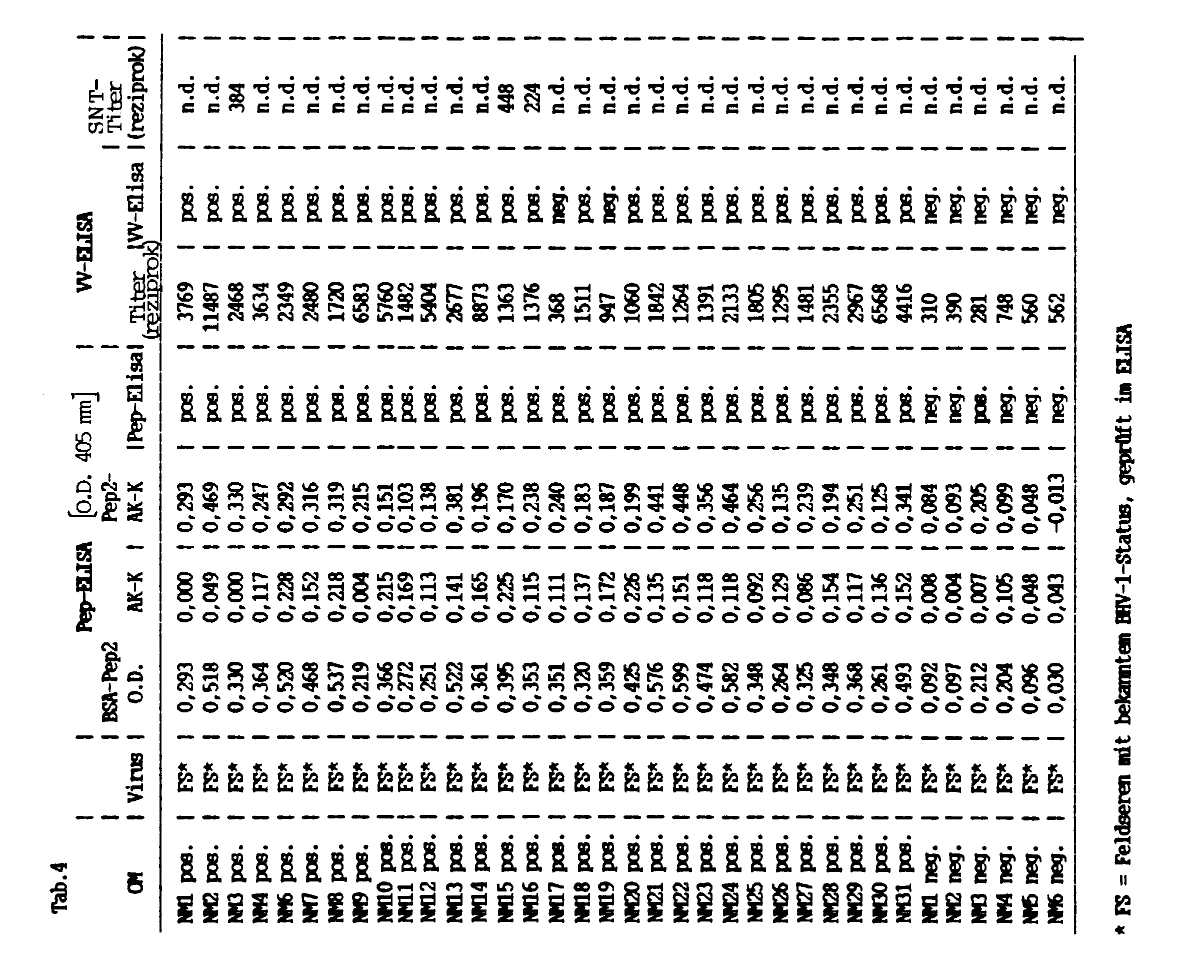

Tab. 5 zeigt die reziproken Antikörpertiter von Rinderseren im SNT nach Immunisierung mit dem Stamm N569.

**Tab.5**

| | Impfmodus | | | |
|---|---|---|---|---|
| Datum | i.m. OM 81 | i.n. OM 83 | i.vag. OM 90 | i.v. OM 89 |
| 11.07 | <2 | <2 | <2 | <2 |
| 12.07 | <2 | <2 | <2 | <2 |
| 13.07 | <2 | <2 | <2 | <2 |
| 14.07 | 2 | <2 | <2 | <2 |
| 15.07 | <2 | <2 | <2 | <2 |
| 16.07 | <2 | <2 | <2 | <2 |
| 17.07 | <2 | <2 | <2 | <2 |
| 18.07 | 3 | <2 | <2 | <2 |
| 19.07 | 2 | <2 | <2 | 3 |
| 20.07 | 2 | <2 | <2 | 6 |
| 21.07 | 4 | 2 | <2 | 23 |
| 22.07 | 3 | 2 | <2 | 45 |
| 23.07 | 4 | 6 | 2 | 90 |
| 24.07 | 5 | 11 | 2 | 128 |
| 25.07 | 8 | 16 | 3 | 64 |
| 27.07 | 3 | 13 | <2 | 152 |
| 30.07 | 9 | 16 | 7 | 152 |
| 01.08 | 11 | 11 | 6 | 128 |
| 03.08 | 13 | 27 | 6 | 181 |
| 06.08 | 27 | 11 | 8 | 107 |
| 08.08 | 45 | 27 | 9 | 152 |

## Patentansprüche

1. Verwendung von BHV-1-Stämmen zur Herstellung von Impfstoffen gegen BHV-1 Infektionen die eine Unterscheidung geimpfter Tiere von feldinfizierten Tieren, erlauben, wobei diese BHV-1-Stämme dadurch gekennzeichnet sind, daß sie
- in ihrem Genom Änderungen im Bereich enthalten, der für Glycoprotein g-IV kodiert,
- diese Änderungen in Bereichen liegen, die für Glycoprotein g-IV nichtessentiell sind,
- diese für Glycoprotein g-IV nichtessentiellen Bereiche Epitope betreffen, die im Glycoprotein g-IV des BHV Stammes Reg. Nr. I 1204 (Institut Pasteur vom 13.4.1992) dem Bereich der Aminosäure-Positionen 310-338 entsprechen,
- in diesen für Glycoprotein g-IV des BHV Stammes Reg. Nr. I 1204 (Institut Pasteur vom 13.4.1992) nichtessentiellen Bereichen im Vergleich zu Feldviren ein oder mehrere Nukleotid(e) ausgetauscht, deletiert oder inseriert sind.

2. Verwendung gemäß Anspruch 1, wobei als BHV-1 Stamm der Stamm Reg. Nr. I 1204 (Institut Pasteur vom 13.4.1992) eingesetzt wird.

3. DNA-Sequenz die für das essentielle Glycoprotein g-IV des BHV-1-Stammes Reg. Nr. I 1204 (Institut Pasteur vom 13.4.1992) kodiert.

4. Protein g-IV des BHV-1-Stammes Reg. Nr. I 1204 (Institut Pasteur vom 13.4.1992).

5. Verwendung von Peptiden die homolog zu Aminosäure-Sequenzen von nichtessentiellen Bereichen in Glycoprotein g-IV in BHV-1 Feldviren sind und die heterolog zu den geänderten Aminosäure-Sequenzen Position 310-338 im Glycoprotein g-IV des BHV-1 Stammes Reg. Nr. I 1204 (Institut Pasteur vom 13.4.1992) sind in serologischen Verfahren zur Unterscheidung von Rindern, die mit BHV-1 Feldviren infiziert sind von Rindern, die mit Impfstoffen auf Basis des BHV-1 Stammes Reg. Nr. I 1204 (Institut Pasteur vom 13.4.1992) geimpft wurden.

## Claims

1. Use of BHV-1 strains for the preparation of vaccines against BHV-1 infections which permit differentiation of vaccinated livestock from field-infected livestock, these BHV-1 strains being characterized in that they
- contain in their genome modifications in the region which codes for glycoprotein g-IV,
- these modifications are located in regions which are nonessential for glycoprotein g-IV,
- these regions which are nonessential for glycoprotein g-IV relate to epitopes which correspond to the region of amino acid positions 310-338 in the glycoprotein g-IV of the BHV strain Reg. No. I 1204 (Institut Pasteur of 13.4.1992),
- one or more nucleotide(s) are exchanged, deleted or inserted, by comparison with field viruses, in these regions which are nonessential for glycoprotein g-IV of the BHV strain Reg. No. I 1204 (Institut Pasteur of 13.4.1992).

2. Use according to Claim 1, where the strain Reg. No. I 1204 (Institut Pasteur of 13.4.1992) is employed as BHV-1 strain.

3. DNA sequence which codes for the essential glycoprotein g-IV of the BHV-1 strain Reg. No. I 1204 (Institut Pasteur of 13.4.1992).

4. Protein g-IV of the BHV-1 strain Reg. No. I 1204 (Institut Pasteur of 13.4.1992).

5. Use of peptides which are homologous to amino acid sequences of nonessential regions in glycoprotein g-IV in BHV-1 field viruses and which are heterologous to the modified amino acid sequences position 310-338 in the glycoprotein g-IV of the BHV-1 strain Reg. No. I 1204 (Institut Pasteur of 13.4.1992), in serological methods for differentiating cattle which are infected with BHV-1 field viruses from cattle which have been immunized with vaccines based on BHV-1 strain Reg. No. I 1204 (Institut Pasteur of 13.4.1992).

## Revendications

1. Utilisation de souches BHV-1 pour la préparation de vaccins contre des infections par BHV-1, qui permettent de faire une distinction entre des animaux vaccinés et des animaux infectés par des virus naturels ou sauvages, ces souches BHV-1 étant caractérisées en ce que
- elles contiennent dans leur génome des modifications dans le domaine qui code pour la glycoprotéine g-IV,
- ces modifications sont situées dans des domaines qui ne sont pas essentiels pour la glycoprotéine g-IV,
- ces domaines non essentiels pour la glycoprotéine g-IV, concernent des épitopes qui correspondent, dans la glycoprotéine g-IV de la souche BHV, numéro matricule I 1204 (Institut Pasteur du 13.4.1992), au domaine des positions d'acides aminés 310-338,
- dans ces domaines non essentiels pour la glycoprotéine g-IV de la souche BHV, numéro matricule I 1204 (Institut Pasteur du 13.4.1992), par rapport à ceux de virus sauvages, un ou plusieurs nucléotides ont été remplacés, supprimés ou insérés.

2. Utilisation selon la revendication 1, dans laquelle on met en oeuvre, comme souche BHV-1, la souche numéro matricule I 1204 (Institut Pasteur du 13.4.1992).

3. Séquence d'ADN qui code pour la glycoprotéine essentielle g-IV de la souche BHV-1 numéro matricule I 1204 (Institut Pasteur du 13.4.1992).

4. Protéine g-IV de la souche BHV-1 numéro matricule I 1204 (Institut Pasteur du 13.4.1992).

5. Utilisation de peptides qui sont homologues vis-à-vis des séquences d'acides aminés de domaines non essentiels dans la glycoprotéine g-IV dans des virus sauvages de type BHV-1 et qui sont hétérologues vis-à-vis des séquences d'acides aminés modifiées aux positions 310-338 dans la glycoprotéine g-IV de la souche BHV-1 numéro matricule I 1204 (Institut Pasteur du 13.4.1992) dans des procédés sérologiques, pour faire une distinction entre des bovins qui ont été infectés par des virus sauvages BHV-1 et des bovins qui ont été vaccinés avec des vaccins à base de la souche BHV-1 numéro matricule I 1024 (Institut Pasteur du 13.4.1992).
